# EUROPEAN PATENT APPLICATION

(11) **EP 4 837 807 A1**
(43) Date of publication of application: **11.11.2026**
(21) Application number: 24919013.3
(22) Date of filing: 19.01.2024
(51) Int. Cl.: B60W 40/08, A61B 5/00, B60W 50/00, G06V 20/59

(54) **APPARATUS AND METHOD FOR INTEGRATING VITAL DATA OF VEHICLE**

(71) Applicant: LG Electronics Inc., Yeongdeungpo-gu Seoul 07336 (KR)
(72) Inventor: PARK, Suho, Seoul 06772 (KR)
(74) Representative: Vossius & Partner Patentanwälte Rechtsanwälte mbB
(86) International application number: PCT/KR2024/095038
(87) International publication number: WO 2025/154989

(57) **Abstract**

An apparatus for integrating vital data of a vehicle according to the present invention comprises: a reception unit for receiving respective pieces of vital data collected by a plurality of cameras installed inside the vehicle; a data determination unit for evaluating the reliability of the received vital data to assign a weight thereto, and selecting or converging the vital data on the basis of the assigned weight; and a data providing unit for providing finally generated vital data so as to estimate a state index of a driver. Accordingly, reliable input data can be provided for high-level vital analysis.

## Description

### Technical Field

The present disclosure relates to a vital data integration device for a vehicle and a vital data integration method for a vehicle and, more particularly, to a vital data integration device and a vital data integration method that use respective vital data items measured by a plurality of cameras within a vehicle.

### Background Art

A vehicle is an apparatus that transports a user in a direction desired by the user. A typical example of a vehicle is an automobile.

A trend has emerged in which vehicles are equipped with various sensors, electronic devices, and equivalents thereof to improve user convenience. Particularly, to enhance user convenience, extensive research has been conducted on Advanced Driver Assistance Systems (ADAS). Furthermore, the development of autonomous vehicles has been actively conducted.

In addition, in recent years, increasing research has been conducted on the technology of continuously monitoring a driver's drowsiness state. In addition, Euro NCAP, which is a European vehicle evaluation organization, is scheduled to introduce, as an evaluation item, the technology of monitoring the degree of drowsiness of a driver and an occupant in a non-contact manner. To this end, a plurality of cameras are used within a vehicle to track a facial expression and a facial surface of a driver without obstruction, thereby monitoring the driver's drowsiness.

### Disclosure of Invention

### Technical Problem

An object of an embodiment of the present disclosure is to provide a vital data integration device for a vehicle and a vital data integration method for a vehicle, the device and the method being capable of selecting a vital data item or fusing vital data items from among a plurality of vital data items acquired using a plurality of cameras within a vehicle.

### Solution to Problems

To this end, a vital data integration device according to an embodiment of the disclosure is capable of selecting a vital data item or fusing vital data items from among vital data items collected by a plurality of cameras installed within a vehicle, thereby estimating a driver's drowsiness in a more reliable manner.

In addition, the vital data integration device for a vehicle is capable of evaluating the respective reliabilities of vital data items on the basis of various collected factors, assigning different weight values on the basis of the result of the evaluation, and generating high-reliability vital data items.

Specifically, the vital data integration device for a vehicle according to the embodiment of the present disclosure may include a reception unit that receives respective vital data items collected by a plurality of cameras installed within the vehicle; a data determination unit that assigns respective weight values to the received vital data items by evaluating the respective reliabilities thereof, and selects a vital data item or fuses vital data items on the basis of the assigned weight values; and a data provision unit that provides a finally generated vital data item to estimate a state index of a driver.

In an embodiment, said evaluating the respective reliabilities of the received vital data items comprises determining different weight values of the received vital data items on the basis of at least one of the following: the respective reliabilities of the plurality of cameras, information about landmarks in images captured by the plurality of cameras, areas occupied by the faces in the images, or the amount of change compared with a previous value of the vital data item.

In an embodiment, the plurality of cameras may be installed around a driver seat within the vehicle and measure a vital data item of the driver using a rPPG (remote PPG) method, and the reception unit may temporally synchronize a plurality of measured vital data items and transfer the resulting vital data items to the data determination unit.

In an embodiment, the data determination unit may remove a vital data item having an abnormal value from among the plurality of vital data items, then evaluate the respective reliabilities of normal vital data items, and assign respective weight values to the normal vital data items.

In an embodiment, the data determination unit may select a vital data item having a high reliability weight on the basis of the assigned weight values or fuse vital data items to which respective reliability weight values are assigned, and transfer the selected vital data item or the vital data item obtained by the fusion to the data provision unit.

In an embodiment, the data determination unit may calculate the average of the respective weight values of the vital data items on the basis of the assigned weight values, select vital data items based on the calculated average, integrate the selected vital data items into a single stream, and transfer the single stream to the data provision unit.

In an embodiment, the data determination unit may apply the assigned weight values to the respective probability values of the vital data items, then calculate an average on the basis of the assigned weight values, and calculate the respective reliability-weighted probabilities of the vital data items.

In an embodiment, the data determination unit may select a vital data item having the highest probability among the reliability-weighted probabilities or generate a vital data item obtained by the fusion by calculating the average of the reliability-weighted probabilities, and transfer the selected vital data item or the vital data item obtained by the fusion to the data provision unit.

In an embodiment, the data determination unit may adjust the respective reliability weight values of the vital data items based on control information of the vehicle and movement information of an occupant.

In an embodiment, the data determination unit may determine, based on the control information of the vehicle, that the respective amounts of change in the respective vital data items have occurred temporarily and, based on the determination, reduce the respective reliability weight values of the vital data items.

In an embodiment, the control information of the vehicle may be a control result that corresponds to any one of the following: braking, starting, or steering activation, each of which exceeds a range set by a driver's operation or an ADAS system.

In an embodiment, the data determination unit may determine that the control information of the vehicle, which is received for a predetermined time or more, remains unchanged, and, based on the determination, increase the respective reliability weight values of the vital data items or shorten the period for calculating the reliability weight values.

In an embodiment, the data determination unit may determine, based on the movement information of the occupant, that the respective amounts of change in the vital data items have occurred temporarily and, based on the determination, reduce the respective reliability weight values of the vital data items or lengthen the period for calculating the reliability weight values.

In addition, a vital data integration method for a vehicle may include: receiving respective vital data items collected by a plurality of cameras installed within the vehicle; assigning respective weight values to the received vital data items by evaluating the respective reliabilities thereof; selecting a vital data item or fusing vital data items on the basis of the assigned weight values; and estimating a state index of a driver by providing a finally generated vital data item as input data.

In an embodiment, the assigning of the respective weight values comprises determining different weight values of the received vital data items on the basis of at least one of the following: the respective reliabilities of the plurality of cameras, information about landmarks in images captured by the plurality of cameras, the areas occupied by the faces in the images, or the amount of change compared with a previous value of the vital data item.

In an embodiment, the assigning of the respective weight values may include additionally adjusting the respective reliability weight values of the vital data items based on control information of the vehicle and movement information of an occupant.

### Advantageous Effects of Invention

The effects of a vital data integration device for a vehicle according to the present disclosure and the effects of a vital data integration method for a vehicle according to the present disclosure are described as follows.

According to an embodiment of the present disclosure, vital data items of the driver or occupant can be collectively provided in a stable, seamless, and highly precise manner when the drowsiness, stress, health information, and the like of a driver or occupant are estimated using a plurality of cameras. That is, the reliable input data items can be provided for high-level analysis of vital data items.

### Brief Description of Drawings

FIG. 1 is a view illustrating a vehicle associated with an embodiment of the present disclosure.
FIG. 2 is a set of views illustrating the vehicle associated with the embodiment of the present disclosure when viewed from various angles.
FIGS. 3 and 4 are views, each illustrating the interior of the vehicle associated with the embodiment of the present disclosure.
FIGS. 5 and 6 are views that are referenced to describe various objects related to the travel of the vehicle associated with the embodiment of the present disclosure.
FIG. 7 is a block diagram that is referenced to a vital data integration device installed in the vehicle associated with the present disclosure
FIG. 8 is a block diagram that is referenced to describe how the vital data integration device according to an embodiment of the present disclosure interacts with other components of the vehicle.
FIG. 9 is a block diagram that is referenced to describe a detailed configuration of the vital data integration device according to the embodiment of the present disclosure.
FIG. 10 is a representative flowchart that is referenced to describe a vital data integration method according to an embodiment of the present disclosure.
FIG. 11 is a view that is referenced to describe the collection of vital data items using a plurality of cameras within the vehicle associated with the embodiment of the present disclosure.
FIG. 12 is an exemplary view that is referenced to describe data selection that uses a hard voting technique according to an embodiment of the present disclosure.
FIGS. 13 and 14 are views that are referenced to describe examples in which a face recognition algorithm according to an embodiment of the present disclosure assigns different weight values to vital data items, respectively.
FIG. 15 is a flowchart that is referenced to describe a method, according to an embodiment of the present disclosure, of generating a final input data item by selecting a vital data item or fusing vital data items via a soft voting technique.
FIGS. 16 and 17 are a block diagram and a flowchart that are referenced to describe a process in which, according to an embodiment of the present disclosure, a plurality of vital data items are acquired, and then vital data items are integrated, or a vital data item is selected to be provided to a state estimation algorithm for a driver.

### Mode for the Invention

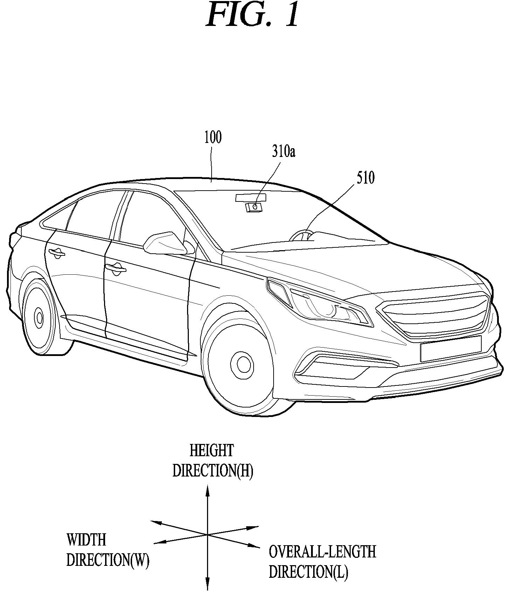

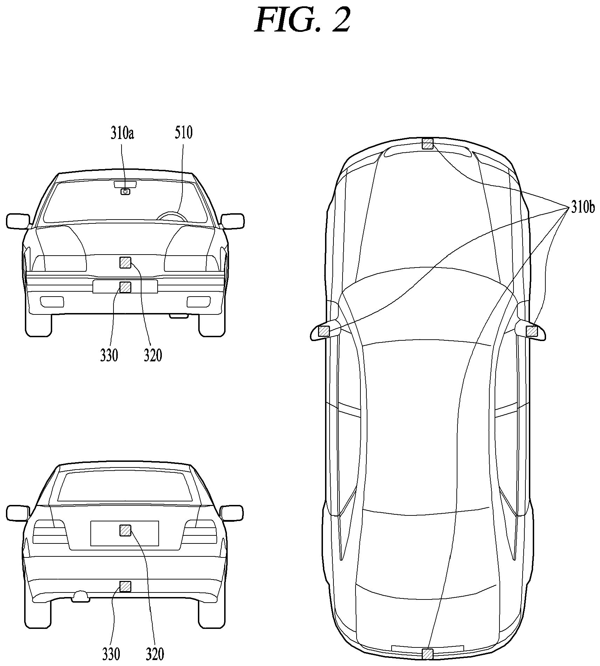

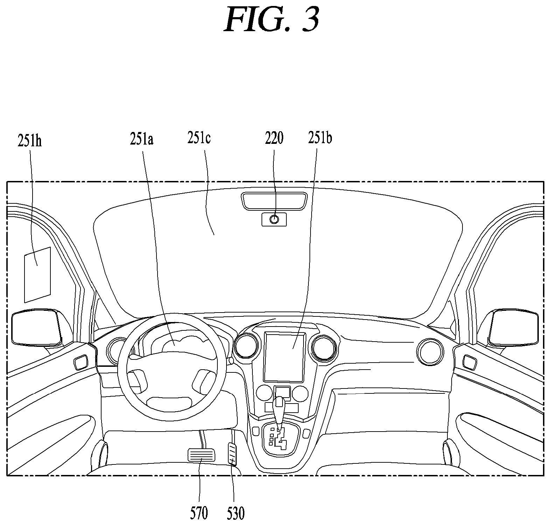

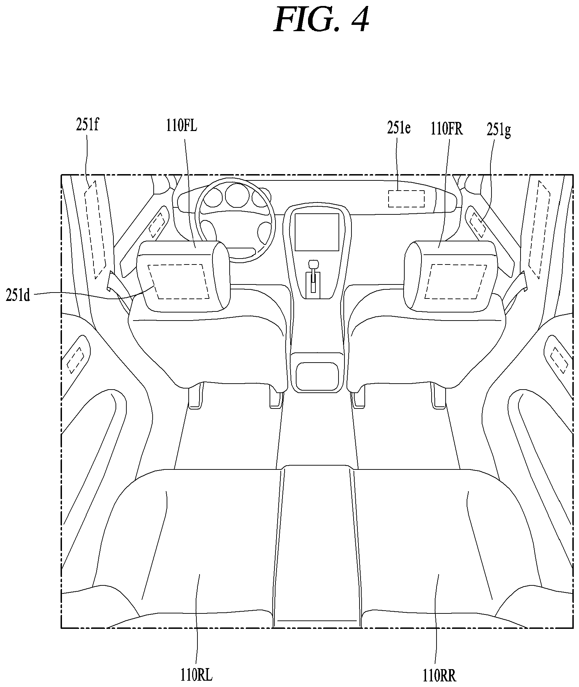

FIGS. 1 and 2 are views each illustrating the exterior of a vehicle associated with an embodiment of the present disclosure. FIGS. 3 and 4 are views each illustrating the interior of the vehicle associated with the embodiment of the present disclosure.

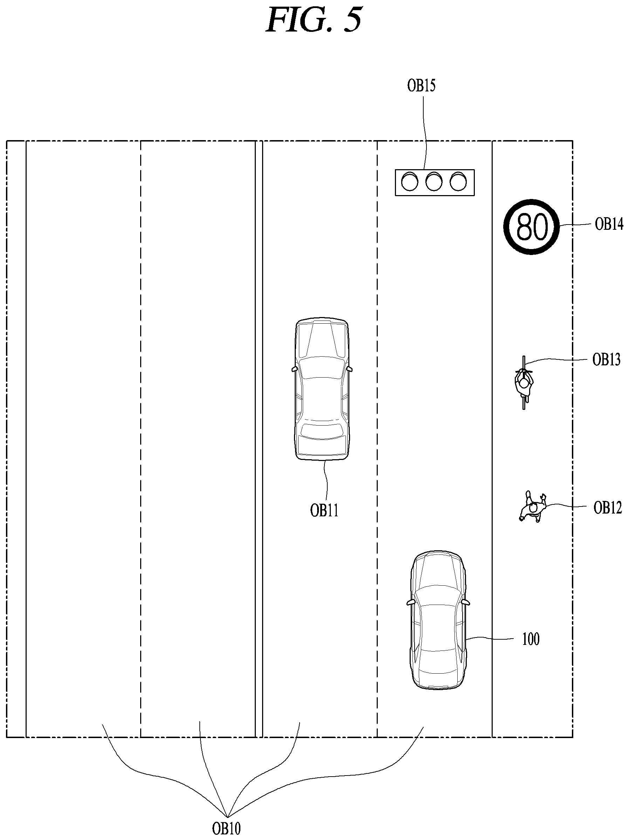

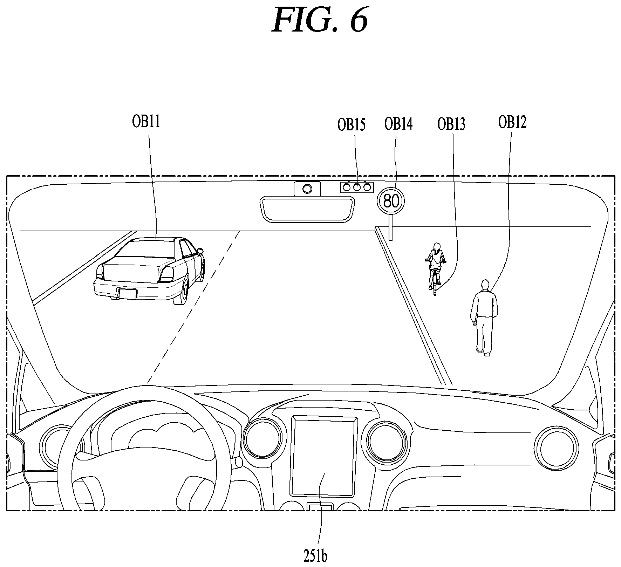

FIGS. 5 and 6 are views each illustrating various objects related to the travel of the vehicle associated with the embodiment of the present disclosure.

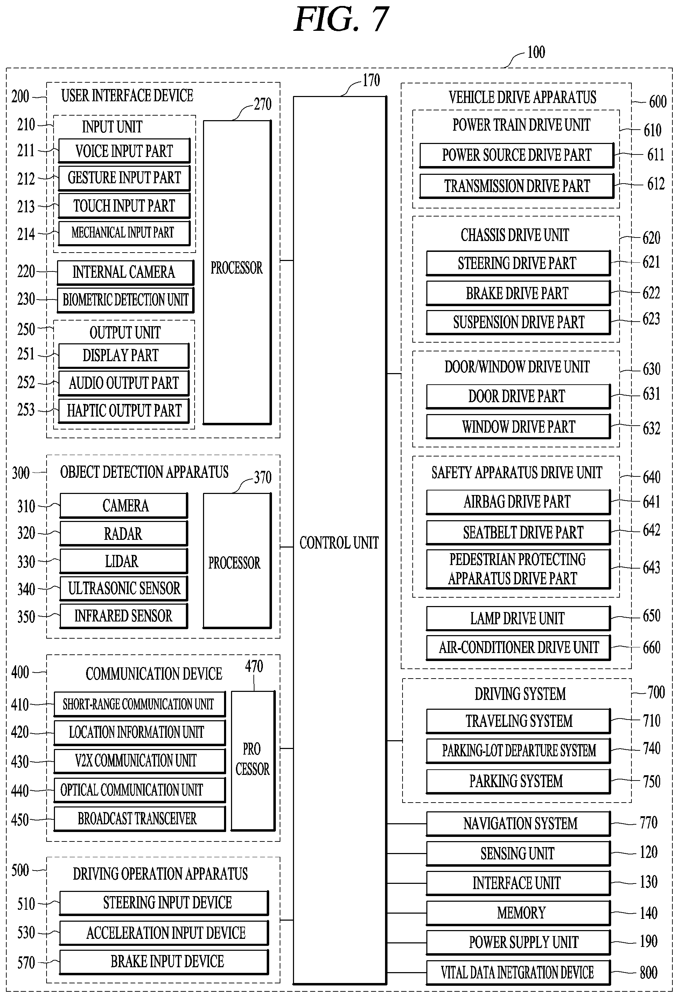

FIG. 7 is a block diagram that is referenced to describe the vehicle associated with the embodiment of the present disclosure.

With reference to FIGS. 1 to 7, a vehicle 100 may include wheels that rotate by a power source and a steering input apparatus 510 for adjusting the travel direction of the vehicle 100.

The vehicle 100 may be an autonomous driving vehicle. The vehicle 100 may switch to an autonomous driving mode or a manual mode in response to a user input. For example, the vehicle 100 may switch from the manual mode to the autonomous driving mode or from the autonomous driving mode to the manual mode in response to the user input received through a user interface device 200 (hereinafter referred to as a 'user terminal').

The vehicle 100 may switch to the autonomous driving mode or the manual mode on the basis of driving situation information. The driving situation information may be generated on the basis of object information provided by an object detection device 300. For example, the vehicle 100 may switch from the manual mode to the autonomous driving mode or from the autonomous driving mode to the manual mode on the basis of the driving situation information generated by the object detection device 300. For example, the vehicle 100 may switch from the manual mode to the autonomous driving mode or from the autonomous driving mode to the manual mode on the basis of the driving situation information received through a communication device 400.

The vehicle 100 may switch from the manual mode to the autonomous driving mode or from the autonomous driving mode to the manual mode on the basis of an information item, a data item, or a signal, each of which is provided by an external device.

In a case where the vehicle 100, which is an autonomous driving vehicle, may be driven in the autonomous driving mode, the vehicle 100 may be driven through the use of an operational system 700. For example, the vehicle 100, which is an autonomous driving vehicle, may be driven on the basis of an information item, a data item, or a signal, each of which is generated by a driving system 710, a parking-lot departure system 740, or a parking system 750.

In a case where the vehicle 100, which is an autonomous driving vehicle, is driven in the manual mode, the vehicle 100 may receive a user input for driving through a driving operation apparatus 500. The vehicle 100 may be driven in response to the user input received through the driving operation apparatus 500.

The overall length refers to the length from the front end to the rear end of the vehicle 100, the width refers to the width of the vehicle 100, and the height refers to the length from the bottom of the wheel to the roof. In the following description, an overall-length direction L may refer to a direction which serves as a reference for measuring the overall length of the vehicle 100, a width direction W may refer to a direction that serves as a reference for measuring the width of the vehicle 100, and a height direction H may refer to a direction that serves as a reference for measuring the height of the vehicle 100.

As illustrated in FIG. 7, the vehicle 100 may include the user interface device (hereinafter referred to as the 'user terminal') 200, the object detection device 300, the communication device 400, the driving operation apparatus 500, a vehicle drive apparatus 600, the operational system 700, a navigation system 770, a sensing unit 120, a vehicular interface unit 130, a memory 140, a control unit 170 and a power supply unit 190.

According to an embodiment, the vehicle 100 may further include one or more constituent elements in addition to the constituent elements described in the present specification or may omit one or more of the described constituent elements.

The user interface device 200 is a device for communication between the vehicle 100 and a user. The user interface device 200 may receive the user input and may provide information, generated by the vehicle 100, to the user. The vehicle 100 may provide a user interface (UI) or user experience (UX) through the user interface device (hereinafter referred to as the 'user terminal') 200.

The user interface device 200 may include an input unit 210, an internal camera 220, a biometric detection unit 230, an output unit 250, and a processor 270. According to an embodiment, the user interface device 200 may further include one or more constituent elements in addition to the constituent elements described in the present specification or may omit one or more of the described constituent elements.

The input unit 210 serves to receive information as input from the user. The processor 270 may analyze data items collected through the input unit 210 and may convert the data items into a control command of the user.

The input unit 210 may be arranged within the vehicle 100. For example, the input unit 210 may be arranged in a region of the steering wheel, a region of the instrument panel, a region of the seat, a region of each pillar, a region of the door, a region of the center console, a region of the headlining, a region of the sun visor, a region of the windshield, a region of the window, or a region of a similar location.

The input unit 210 may include a voice input part 211, a gesture input part 212, a touch input part 213, and a mechanical input part 214.

The voice input part 211 may convert a user's voice input into an electrical signal. The electrical signal, resulting from the conversion, may be provided to the processor 270 or the control unit 170. The voice input part 211 may include one or more microphones.

The gesture input part 212 may convert a user's gesture input into an electrical signal. The electrical signal, resulting from the conversion, may be provided to the processor 270 or the control unit 170.

The gesture input part 212 may include at least one of the following: an infrared sensor or an image sensor, each of which serves to detect the user's gesture input. According to an embodiment, the gesture input part 212 may detect a user's three-dimensional (3D) gesture input. To this end, the gesture input part 212 may include a plurality of light-emitting units, each emitting an infrared ray, or a plurality of image sensors.

The gesture input part 212 may detect the user's 3D gesture input through a time-of-flight (TOF) technique, a structured light technique, or a disparity technique.

The touch input part 213 may convert the user's touch input into an electrical signal. The electrical signal, resulting from the conversion, may be provided to the processor 270 or the control unit 170.

The touch input part 213 may include a touch sensor for detecting the user's touch input. According to an embodiment, the touch input part 213 may be integrally formed with a display part 251, thereby forming a touch screen. The touch screen may provide both an input interface and an output interface between the vehicle 100 and the user.

The mechanical input part 214 may include at least one of the following: a button, a dome switch, a jog wheel, or a jog switch. An electrical signal generated by the mechanical input part 214 may be provided to the processor 270 or the control unit 170. The mechanical input part 214 may be arranged on the steering wheel, the center fascia, the center console, the cockpit module, the door, or an equivalent thereof.

The internal camera 220 may acquire an image of the interior of the vehicle 100. The processor 270 may detect the user's state on the basis of the image of the interior of the vehicle 100. The processor 270 may acquire the user's gaze information from the image of the interior of the vehicle 100. The processor 270 may detect the user's gesture from the image of the interior of the vehicle 100.

The biometric detection unit 230 may acquire the user's biometric information. The biometric detection unit 230 may include a sensor for acquiring the user's biometric information and may acquire the user's fingerprint information, heart rate information, and equivalents thereof using the sensor. The biometric information may be used for user authentication.

The output unit 250 may generate outputs associated with a visual sense, an auditory sense, a haptic sense, and the like. The output unit 250 may include at least one of the following: the display part 251, an audio output part 252, or a haptic output part 253.

The display part 251 may display graphic objects that correspond to various pieces of information, respectively. The display part 251 may include at least one of the following: a liquid crystal display (LCD), a thin-film transistor-LCD (TFT LCD), an organic light-emitting diode (OLED), a flexible display, a three-dimensional (3D) display, or an E-Ink display.

The display part 251 may be configured to have a layered structure or an integrated structure with the touch input part 213, thereby forming a touch screen.

The display part 251 may be configured as a head-up display (HUD). In a case where the display part 251 may be configured as the HUD, the display part 251 may be equipped with a projection module and thus may output information through an image that is projected onto the window shield or the window.

The display part 251 may be a transparent display. The transparent display may be attached to the windshield or the window. The transparent display may have a predetermined degree of transparency and may display a predetermined screen thereon. To exhibit a degree of transparency, the transparent display may include at least one of the following: a thin-film electroluminescent (TFEL), a transparent organic light-emitting diode (OLED), a transparent liquid crystal display (LCD), a transmissive transparent display, or a transparent light-emitting diode (LED) display. The transparent display may have an adjustable level of transparency.

The user interface device 200 may include a plurality of display parts, for example, display parts 251a to 251g.

The display part 251 may be arranged in a region of the steering wheel, a region 521a, 251b, or 251e of the instrument panel, a region 251d of the seat, a region 251f of each pillar, a region 251g of the door, a region of the center console, a region of the headlining, or a region of the sun visor, or may be formed in a region 251c of the windshield or a region of the window.

The audio output part 252 converts an electrical signal, provided from the processor 270 or the control unit 170, into an audio signal and outputs the audio signal. To this end, the audio output part 252 may include at least one speaker.

The haptic output part 253 generates a tactile output. For example, the haptic output part 253 may operate to vibrate a steering wheel, a safety belt, and seats 110FL, 110FR, 110RL, and 110RR, thereby enabling the user to perceive a vibration output.

The processor (hereinafter referred to as a 'control unit') 270 may control the overall operation of each unit of the user interface device 200. According to an embodiment, the user interface device 200 may include a plurality of processors 270 or may be configured without including the processor 270.

In a case where the processor 270 is not included in the user interface device 200, the user interface device 200 may operate under the control of a processor of another apparatus within the vehicle 100 or under the control of the control unit 170.

The user interface device 200 may be referred to as a vehicular display device. The user interface device 200 may operate under the control of the control unit 170.

The object detection device 300 is a device for detecting an object located outside the vehicle 100. This object may be an external entity associated with the operation of the vehicle 100. With reference to FIGS. 5 and 6, examples of an object O may include a lane OB10, a different vehicle OB11, a pedestrian OB12, a two-wheeled vehicle OB13, traffic signals OB14 and OB15, ambient light, a road, a structure, a speed bump, a terrain feature, an animal, and the like.

The lane OB10 may be a travel lane, a lane adjacent to the travel lane, or a lane along which a different vehicle in the opposite direction travels. The lane OB10 may be conceptually represented as including the left and right boundary lines that form the lane.

The different vehicle OB11 may be a vehicle that travels in the vicinity of the vehicle 100. The different vehicle OB11 may be a vehicle located within a predetermined distance from the vehicle 100. For example, the different vehicle OB11 may be a vehicle that travels ahead of or behind the vehicle 100.

The pedestrian OB12 may be a person located in the vicinity of the vehicle 100. The pedestrian OB12 may be a person located within a predetermined distance from the vehicle 100. For example, the pedestrian OB12 may be a person located on a sidewalk or roadway.

The two-wheeled vehicle OB12 may be a person-carrying vehicle that is located in the vicinity of the vehicle 100 and moves on two wheels. The two-wheeled vehicle OB12 may be a person-carrying vehicle that is located within a predetermined distance from the vehicle 100 and has two wheels. For example, the two-wheeled vehicle OB13 may be a motorcycle or a bicycle that is located on a sidewalk or roadway.

Examples of the traffic signal may include the traffic sign OB14, the traffic light OB15, or a pattern or text drawn on a road surface.

The light may be light generated from a lamp provided on a different vehicle. The light may be light generated from a street lamp. The light may be sunlight.

The road may include a road surface, a curved section, an upward slope, a downward slope, and the like.

The structure may be an object that is located in the vicinity of a road and fixed to the ground. Examples of the structure may include a street lamp, a roadside tree, a building, an electric pole, a traffic light, and a bridge.

Examples of the terrain feature may include a mountain, a hill, and the like.

The objects may be classified into movable objects and stationary objects. Examples of the movable objects may be conceptually represented as including another vehicle and a pedestrian. Examples of the stationary objects may be conceptually represented as including a traffic light, a road, and a structure.

The object detection device 300 may include a camera 310, a radar 320, a LiDAR 330, an ultrasonic sensor 340, an infrared sensor 350, and a processor 370.

According to an embodiment, the object detection device 300 may further include one or more constituent elements in addition to the constituent elements described in the present specification or may omit one or more of the described constituent elements.

The camera 310 may be located on an appropriate portion of the exterior of the vehicle to capture an image of the surroundings of the vehicle 100. The camera 310 may be a monocular camera, a stereo camera 310a, an around-view monitoring (AVM) camera 310b, or a 360-degree camera.

For example, the camera 310 may be arranged adjacent to a front windshield within the vehicle to acquire an image of the surroundings in front of the vehicle. Alternatively, the camera 310 may be arranged in the vicinity of the front bumper or the radiator grill.

For example, the camera 310 may be arranged adjacent to a rear glass pane within the vehicle to capture the image of the surroundings behind the vehicle. Alternatively, the camera 310 may be arranged adjacent to the rear bumper, the trunk, or the tailgate.

For example, the camera 310 may be arranged adjacent to at least one of the side windows within the vehicle to capture an image of the surroundings alongside the vehicle. Alternatively, the camera 310 may be arranged in the vicinity of the side mirror, the fender, or the door.

The camera 310 may provide an acquired image to the processor 370.

The radar 320 may include an electromagnetic wave transmission unit and an electromagnetic wave reception unit. The radar 320 may be configured based on a pulse radar method or a continuous wave radar method, in accordance with the principle of emitting a radio wave. The radar 320 may be configured based on a Frequency Modulated Continuous Wave (FMCW) method or a Frequency Shift Keying (FSK) method, each of which is determined according to a signal waveform, among continuous wave radar methods.

The radar 320 may detect an object based on a time-of-flight (TOF) technique or a phase-shift technique through the use of electromagnetic waves and may determine the location of the detected object, the distance to the detected object, and the relative speed with respect to the detected object.

The radar 320 may be arranged at an appropriate position on the exterior of the vehicle 100 to detect an object that is located in front of, behind, or alongside the vehicle 100.

The LiDAR 330 may include a laser transmission unit and a laser reception unit. The LiDAR 330 may be configured based on the time-of-flight (TOF) technique or the phase-shift technique.

The LiDAR 330 may be configured as a drive-type or non-drive-type LiDAR.

In a case where the LiDAR 330 may be configured as a drive-type LiDAR, the LiDAR 330 may be rotated by a motor and may detect an object in the vicinity of the vehicle 100.

In a case where the LiDAR 330 may be configured as a non-drive-type LiDAR, the LiDAR 330 may detect, through optical, an object located within a predetermined range of the vehicle 100. The vehicle 100 may include a plurality of non-drive-type LiDAR's 330.

The LiDAR 330 may detect an object based on the time-of-flight (TOF) technique or the phase-shift technique through the use of laser light and may determine the location of the detected object, the distance to the detected object, and the relative speed with respect to the detected object.

The radar 330 may be arranged at an appropriate position on the exterior of the vehicle 100 to detect an object that is located in front of, behind, or alongside the vehicle 100.

The ultrasonic sensor 340 may include an ultrasonic wave transmission unit and an ultrasonic wave reception unit. The ultrasonic sensor 340 may detect an object through the use of ultrasonic waves and may determine the location of the detected object, the distance to the detected object, and the relative speed with respect to the detected object.

The ultrasonic sensor 340 may be arranged at an appropriate position on the exterior of the vehicle 100 to detect an object located in front of, behind, or alongside the vehicle 100.

The infrared sensor 350 may include an infrared light transmission unit and an infrared light reception unit. The infrared sensor 340 may detect an object through the use of infrared light and may determine the location of the detected object, the distance to the detected object, and the relative speed with respect to the detected object.

The infrared sensor 350 may be arranged at an appropriate position on the exterior of the vehicle 100 to detect an object located in front of, behind, or alongside the vehicle 100.

The processor 370 may control the overall operation of each unit of the object detection device 300.

The processor 370 may detect and track an object on the basis of an acquired image. The processor 370 may perform operations, such as calculation of the distance to the object and calculation of the relative speed with respect to the object, through the use of an image processing algorithm.

The processor 370 may detect and track an object through the use of reflected electromagnetic waves, resulting from the reflection of emitted electromagnetic waves by the object. The processor 370 may perform operations, such as calculation of the distance to the object and calculation of the relative speed with respect to the object, through the use of electromagnetic waves.

The processor 370 may detect and track an object through the use of reflected laser beams, resulting from the reflection of emitted lasers by the object. The processor 370 may perform operations, such as calculation of the distance to the object and calculation of the relative speed with respect to the object, through the use of laser beams.

The processor 370 may detect and track an object through the use of reflected ultrasonic waves, resulting from the reflection of emitted ultrasonic waves by the object. The processor 370 may perform operations, such as calculation of the distance to the object and calculation of the relative speed with respect to the object, through the use of ultrasonic waves.

The processor 370 may detect and track an object through the use of reflected infrared light, resulting from the reflection of emitted infrared light by the object. The processor 370 may perform operations, such as calculation of the distance to the object and calculation of the relative speed with respect to the object, through the use of infrared light.

According to an embodiment, the object detection device 300 may include a plurality of processors 370 or may be configured without including the processor 370. The camera 310, the radar 320, the LiDAR 330, the ultrasonic sensor 340, and the infrared sensor 350 may include respective processors for individual operation.

In a case where the processor 370 is not included in the object detection device 300, the object detection device 300 may operate under the control of a processor of an apparatus within the vehicle 100 or under the control of the control unit 170.

The object detection device 300 may operate under the control of the control unit 170.

The communication device 400 is a device for performing communication with an external device. The external device here may be another vehicle, a mobile terminal, or a server.

To perform communication, the communication device 400 may include a transmission antenna, a reception antenna, and at least one of the following: a radio frequency (RF) circuit or an RF device, each of which is capable of implementing various communication protocols.

The communication device 400 may include a short-range communication unit 410, a location information unit 420, a V2X communication unit 430, an optical communication unit 440, a broadcast transceiver 450, and a processor 470.

According to an embodiment, the communication device 400 may further include one or more constituent elements in addition to the constituent elements in the present specification or may omit one or more of the described constituent elements.

The short-range communication unit 410 is a unit for short-range communication. The short-range communication unit 410 may support short-range communication using at least one of the following technologies: Bluetooth^{TM}, Radio Frequency IDentification (RFID), Infrared Data Association (IrDA), Ultra-Wide Band (UWB), Zig Bee, Near Field Communication (NFC), Wireless-Fidelity (Wi-Fi), Wi-Fi Direct, or Wireless Universal Serial Bus (USB).

The short-range communication unit 410 may form short-range wireless area networks and may perform short-range communication between the vehicle 100 and at least one external device.

The location information unit 420 is a unit for acquiring location information of the vehicle 100. For example, the location information unit 420 may include a Global Positioning System (GPS) module or a Differential Global Positioning System (DGPS) module.

The V2X communication unit 430 is a unit for performing wireless communication with a server (Vehicle to Infrastructure (V2I)), with another vehicle (Vehicle to Vehicle (V2V)), or with a pedestrian (Vehicle to Pedestrian (V2P)). The V2X communication unit 430 may include an RF circuit capable of implementing protocols for communication with an infrastructure (V2I), communication between vehicles (V2V), and communication with a pedestrian (V2P).

The optical communication unit 440 is a unit for performing communication with an external device through the use of light. The optical communication unit 440 may include an optical transmission part for converting an electrical signal into an optical signal and transmitting the optical signal to the outside, and an optical reception part for converting a received optical signal into an electrical signal.

According to an embodiment, the optical transmission part may be integrally formed with a lamp included in the vehicle 100.

The broadcast transceiver 450 is a unit for receiving a broadcast signal from an external broadcast management server or transmitting a broadcast signal to the broadcast management server over a broadcast channel. The broadcast channels may include a satellite channel and a terrestrial channel. The broadcast signals may include a TV broadcast signal, a radio broadcast signal, and a data broadcast signal.

The processor 470 may control the overall operation of each unit of the communication device 400.

According to an embodiment, the communication device 400 may include a plurality of processors 470 or may be configured without including the processor 470.

In a case where the processor 470 is not included in the communication device 400, the communication device 400 may operate under the control of a processor of another apparatus within the vehicle 100 or under the control of the control unit 170.

The communication device 400, together with the user interface device 200, may constitute a vehicular display device. In this case, the vehicular display device may be referred to as a telematics device or an Audio Video Navigation (AVN) device.

The communication device 400 may operate under the control of the control unit 170.

The driving operation apparatus 500 is an apparatus that receives a user input for driving.

In the manual mode, the vehicle 100 may be driven on the basis of a signal provided by the driving operation apparatus 500.

The driving operation apparatus 500 may include the steering input device 510, an acceleration input device 530, and a brake input device 570.

The steering input device 510 may receive an input regarding the travel direction of the vehicle 100 from the user. The steering input device 510 is preferably formed in the form of a wheel, thereby enabling a steering input through the rotation of the wheel. According to an embodiment, the steering input device may also be formed in the shape of a touch screen, a touchpad, or a button.

The acceleration input device 530 may receive, from the user, an input for accelerating the vehicle 100. The brake input device 570 may receive, from the user, an input for decelerating the vehicle 100. The acceleration input device 530 and the brake input device 570 are preferably formed in the shape of a pedal. According to an embodiment, the acceleration input device or the brake input device may also be formed in the shape of a touch screen, a touchpad, or a button.

The driving operation apparatus 500 may operate under the control of the control unit 170.

The vehicle drive apparatus 600 is an apparatus that electrically controls the operations of various apparatuses within the vehicle 100.

The vehicle drive apparatus 600 may include a power train drive unit 610, a chassis drive unit 620, a door/window drive unit 630, a safety apparatus drive unit 640, a lamp drive unit 650, and an air-conditioner drive unit 660.

According to an embodiment, the vehicle drive apparatus 600 may further include one or more constituent elements in addition to the constituent elements described in the present specification or may omit one or more of the described constituent elements.

The vehicle drive apparatus 600 may include a processor. Units of the vehicle drive apparatus 600 may include respective processors for individual operation.

The power train drive unit 610 may control the operation of a power train apparatus.

The power train drive unit 610 may include a power source drive part 611 and a transmission drive part 612.

The power source drive part 611 may execute control of a power source of the vehicle 100.

For example, in a case where the power source of the vehicle is a fossil-fuel-based engine, the power source drive part 610 may execute electronic control of the engine. Accordingly, output torque and other parameters of the engine may be controlled. The power source drive part 611 may adjust the engine output torque under the control of the control unit 170.

For example, in a case where the power source of the vehicle is an electric-energy-based motor, the power source drive part 610 may execute control of the motor. The power source drive part 610 may adjust the rotational speed, torque, and other parameters of the motor under the control of the control unit 170.

The transmission drive part 612 may execute control of the transmission. The transmission drive gearbox part 612 may adjust the state of the transmission. The transmission drive part 612 may change the state of the transmission to Drive (D), Reverse (R), Neutral (N), or Park (P).

In a case where the power source of the vehicle is an engine, the transmission drive part 612 may adjust the engaged state of a gear in Drive (D).

The chassis drive unit 620 may control the operation of a chassis apparatus. The chassis drive unit 620 may include a steering drive part 621, a brake drive part 622, and a suspension drive part 623.

The steering drive part 621 may execute electronic control of a steering apparatus within the vehicle 100. The steering drive part 621 may change the travel direction of the vehicle.

The brake drive part 622 may execute electronic control of a brake apparatus within the vehicle 100. For example, the brake drive part 622 may reduce the speed of the vehicle 100 by controlling the operation of the brakes provided on the wheels.

The brake drive part 622 may individually control each of the plurality of brakes. The brake drive part 622 may control the application of varying braking forces to the plurality of wheels.

The suspension drive part 623 may execute electronic control of a suspension apparatus within the vehicle 100. For example, in a case where a road surface is uneven, the suspension drive part 623 may reduce vibration of the vehicle 100 by controlling the suspension apparatus. The suspension drive part 623 may control each of the plurality of suspensions individually.

The door/window drive unit 630 may execute electronic control of a door apparatus or a window apparatus within the vehicle 100.

The door/window drive unit 630 may include a door drive part 631 and a window drive part 632.

The door drive part 631 may execute control of the door apparatus. The door drive part 631 may control the opening or closing of a plurality of doors included in the vehicle 100. The door drive part 631 may control the opening or closing of the trunk or the tailgate. The door drive part 631 may control the opening or closing of the sunroof.

The window drive part 632 may execute electronic control of the window apparatus. The window drive part 632 may control the opening or closing of a plurality of windows included in the vehicle 100.

The safety apparatus drive unit 640 may execute electronic control of various safety apparatuses within the vehicle 100.

The safety apparatus drive unit 640 may include an airbag drive part 641, a seat belt drive part 642, and a pedestrian protection apparatus drive part 643.

The airbag drive part 641 may execute electronic control of an airbag apparatus within the vehicle 100. For example, when a risk is detected, the airbag drive part 641 may control an airbag in such a manner that the airbag is deployed.

The seat belt drive part 642 may execute electronic control of a seat belt apparatus within the vehicle 100. For example, when a risk is detected, the seat belt drive part 642 may secure occupants seated in seats 110FL, 110FR, 110RL, and 110RR by tightening respective seat belts.

The pedestrian protection apparatus drive part 643 may execute electronic control of the hood lift and the pedestrian airbag. For example, when a collision with a pedestrian is detected, the pedestrian protection apparatus drive part 643 may control the hood and the pedestrian airbag in such a manner that the hood is lifted up and that the pedestrian airbag is deployed.

The lamp drive part 650 may execute electronic control of various lamp apparatuses within the vehicle 100.

The air-conditioner drive unit 660 may execute electronic control of an air conditioner within the vehicle 100. For example, when the temperature within the vehicle 100 is high, the air-conditioner drive unit 660 may control the air conditioner to operate in such a manner as to supply cool air into the vehicle 100.

The vehicle drive apparatus 600 may include a processor. Units of the vehicle drive apparatus 600 may include respective processors for individual operation.

The vehicle drive apparatus 600 may operate under the control of the control unit 170.

The operational system 700 is a system that controls various driving functions of the vehicle 100. The operational system 700 may operate in the autonomous driving mode.

The operational system 700 may include the driving system 710, the parking-lot departure system 740, and the parking system 750.

According to an embodiment, the operational system 700 may further include one or more constituent elements in addition to the constituent elements described in the present disclosure or may omit one or more of the described constituent elements.

The operational system 700 may include a processor. Units of the operational system 700 may include respective processors for individual operation.

According to an embodiment, in a case where the operational system 700 may be implemented in software, the operational system 700 may also conceptually operate at a lower level than the control unit 170.

According to an embodiment, the operational system 700 may conceptually include at least one of the following: the user interface device 200, the object detection device 300, the communication device 400, the vehicle drive apparatus 600, or the control unit 170.

The driving system 710 may enable the vehicle 100 to travel.

The driving system 710 may receive navigation information from the navigation system 770, may provide a control signal to the vehicle drive apparatus 600, and may enable the vehicle 100 to travel. The driving system 710 may receive object information from the object detection device 300, may provide a control signal to the vehicle drive apparatus 600, and may enable the vehicle 100 to travel. The driving system 710 may receive a signal from an external device through the communication device 400, may provide a control signal to the vehicle drive apparatus 600, and may enable the vehicle 100 to travel.

The parking-lot departure system 740 may perform a maneuver to guide the vehicle 100 out of a parking lot.

The parking-lot departure system 740 may receive the navigation information from the navigation system 770, may provide a control signal to the vehicle drive apparatus 600, and may perform a maneuver to guide the vehicle 100 out of the parking lot. The parking-lot departure system 740 may receive object information from the object detection device 300, may provide a control signal to the vehicle drive apparatus 600, and may perform a maneuver to guide the vehicle 100 out of the parking lot. The parking-lot departure system 740 may receive a signal from an external device through the communication device 400, may provide a control signal to the vehicle drive apparatus 600, and may perform a maneuver to guide the vehicle 100 out of the parking lot.

The parking system 750 may perform a maneuver to park the vehicle 100.

The parking system 750 may receive the navigation information from the navigation system 770, may provide a control signal to the vehicle drive apparatus 600, and may perform a maneuver to park the vehicle 100. The parking system 750 may receive object information from the object detection device 300, may provide a control signal to the vehicle drive apparatus 600, and may perform a maneuver to park the vehicle 100. The parking system 750 may receive a signal from an external device through the communication device 400, may provide a control signal to the vehicle drive apparatus 600, and may perform a maneuver to park the vehicle 100.

The navigation system 770 may provide the navigation information. The navigation information may include at least one of the following: map information, information regarding a preset destination, path information based on the preset destination, information about various objects on a path, lane information, or current vehicular location information.

The navigation system 770 may include a memory and a processor. The navigation information may be stored in the memory. The processor may control the operation of the navigation system 770.

According to an embodiment, the navigation system 770 may update previously stored information by receiving information from an external device through the communication device 400.

According to an embodiment, the navigation system 770 may also be classified as a sub-constituent element of the user interface device 200.

The sensing unit 120 may sense the state of the vehicle. The sensing unit 120 may include a posture sensor (e.g., a yaw sensor, a roll sensor, or a pitch sensor), a collision sensor, a wheel sensor, a speed sensor, a tilt sensor, a weight-detection sensor, a heading sensor, a yaw sensor, a gyro sensor, a position module, a vehicle forward/backward movement sensor, a battery sensor, a fuel sensor, a tire sensor, a steering sensor sensing the turning of a steering wheel, an in-vehicle temperature sensor, an in-vehicle humidity sensor, an ultrasonic sensor, an illumination sensor, an accelerator position sensor, a brake pedal position sensor, and other sensors.

The sensing unit 120 may acquire vehicular posture information, vehicular collision information, vehicular direction information, vehicular location information (GPS information), vehicular angle information, vehicular speed information, vehicular acceleration information, vehicular tilt information, vehicular forward/backward movement information, battery information, fuel information, tire information, vehicular lamp information, in-vehicle temperature information, and in-vehicle humidity information. Furthermore, the sensing unit 120 may acquire signals that result from sensing a steering wheel rotation angle, vehicle exterior illumination, pressure applied to an acceleration pedal, pressure applied to a brake pedal, and equivalents thereof.

The sensing unit 120 may further include an acceleration pedal sensor, a pressure sensor, an engine speed sensor, an air flow sensor (AFS), an air temperature sensor (ATS), a water temperature sensor (WTS), a throttle position sensor (TPS), a TDC sensor, a crank angle sensor (CAS), and other sensors.

The vehicular interface unit 130 may serve as an interface for various types of external devices connected to the vehicle 100. For example, the vehicular interface unit 130 may include a port that enables a connection to a mobile terminal and may be connected to the mobile terminal through the port. In this case, the vehicular interface unit 130 may exchange data items with the mobile terminal.

The vehicular interface unit 130 may serve as an interface for supplying electrical energy to the connected mobile terminal. In a case where the mobile terminal is electrically connected to the vehicular interface unit 130, the vehicular interface unit 130 may supply electrical energy, supplied from the power supply unit 190, to the mobile terminal under the control of the control unit 170.

The memory 140 is electrically connected to the control unit 170. Basic data for the units, control data items for controlling operations of the units, and input and output data items may be stored in the memory 140. Examples of the memory 140 may include various hardware storage devices, such as ROM, RAM, EPROM, flash memory, and a hard disk drive. Programs and equivalents thereof for processing or control by the control unit 170 and various data items for the overall operation of the vehicle 100 may be stored in the memory 140.

According to an embodiment, the memory 140 may be integrally formed with the control unit 170 or may be configured as a sub-constituent element of the control unit 170.

The control unit 170 may control the overall operation of each unit within the vehicle 100. The control unit 170 may be referred to as an Electronic Control Unit (ECU).

The power supply unit 190 may supply power necessary for the operation of each constituent element under the control of the control unit 170. Specifically, the power supply unit 190 may be supplied with power from the battery within the vehicle 100 or from other sources.

At least one processor and at least one control unit 170, each of which is included in the vehicle 100, may be configured based on at least one of the following: application-specific integrated circuits (ASICs), digital signal processors (DSPs), digital signal processing devices (DSPDs), programmable logic devices (PLDs), field-programmable gate arrays (FPGAs), processors, controllers, microcontrollers, microprocessors, or electric units performing a similar function.

In embodiments of the present disclosure, the terms 'driver,' 'occupant,' and 'driver or occupant (driver/occupant)' are defined to mean a driver, a passenger, a fellow passenger, an occupant, and the like who are capable of personal driving control according to an embodiment of the present disclosure. In addition, in the present specification, the term 'driver' is defined to include an occupant seated in a driver's seat during autonomous driving of the vehicle.

In addition, in embodiments of the present disclosure, the term 'vehicle' is defined to mean a vehicle that travels according to a driver's operation or a vehicle that is configured to receive an autonomous driving service.

As illustrated in FIG. 7, a vital data integration device 800 according to an embodiment of the present disclosure may be provided in the vehicle 100.

In an embodiment, the vital data integration device 800 may take the form of a service platform providing a vital data integration service. In this case, the vehicle 100 may be provided with a vital data integration service from an associated platform through a network or the like.

The vital data integration device 800 receives a plurality of vital data items of a driver or occupant collected through the plurality of cameras installed at different positions, respectively, within the vehicle 100. The vital data integration device 800 may receive a plurality of temporally synchronized vital data items as a single serialized stream.

The vital data integration device 800 may evaluate the respective reliabilities of the plurality of received vital data items based on a preset reference and accordingly assign different weight values to the plurality of received vital data items, respectively. In this case, the preset reference may be established on the basis of one or more of the following: the performance of a plurality of cameras, the result of executing a face recognition algorithm in each of the plurality of cameras, and the performance of a state estimation algorithm configured to be executed.

The vital data integration device 800 may select a vital data item or fuse vital data items on the basis of the assigned weight values, thereby ultimately generating an input data item for high-level vital data analysis. In this case, input data for high-level vital data analysis may refer to an input value of an algorithm for estimating a state index of the driver or occupant.

The vital data integration device 800 may provide a finally generated input data item to a state estimation algorithm for the driver or occupant, thereby enabling the acquisition of a high-reliability state index result. In this case, the state estimation algorithm for the driver or occupant may include, for example, a drowsiness estimation algorithm, a stress evaluation algorithm, and an emergency state estimation (evaluation) algorithm.

In this manner, the vital data integration device 800 receives a plurality of vital data items of the driver or occupant onboard the vehicle 100, acquired from various angles, evaluates the respective reliabilities of the plurality of received vital data items, and assigns respective weight values to the plurality of evaluated vital data items. Then, the vital data integration device 800 selects a vital data item or fuses vital data items on the basis of the respective weight values, thereby providing a high-reliability input value to the state estimation algorithm.

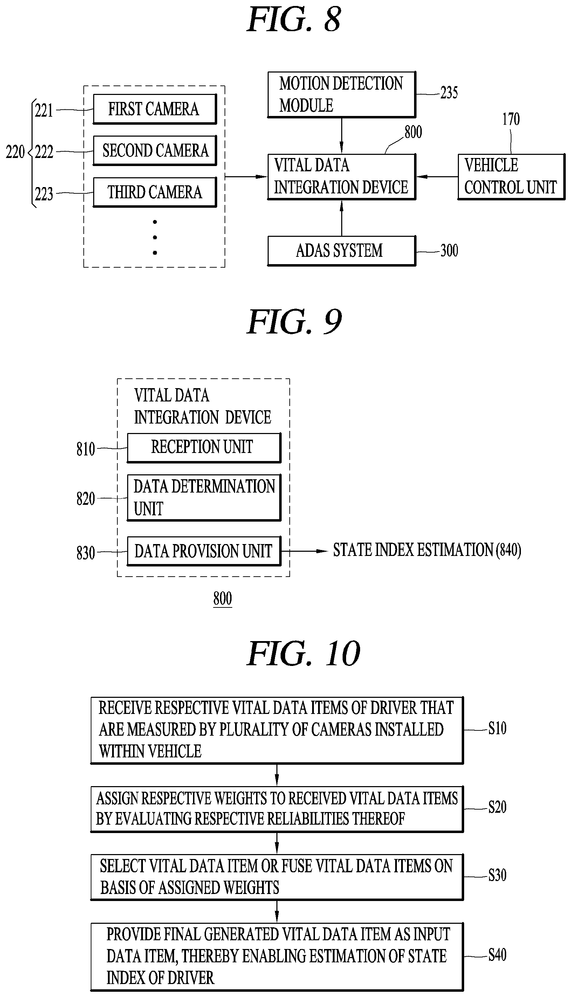

FIG. 8 is a block diagram that is referenced to describe how the vital data integration device 800 according to an embodiment of the present disclosure interacts with other components of the vehicle.

The vital data integration device 800 may communicate with a plurality of cameras 220, a motion detection module 235, a vehicle control unit 170, and an ADAS system 300 within the vehicle. The vital data integration device 800 communicates with these components, thereby acquiring the plurality of vital data items of the driver or occupant. In addition, the vital data integration device 800 communicates with the components to evaluate the respective reliabilities of the plurality of acquired vital data items and to reflect detection results and/or control results of the components in the reliability evaluation.

The vital data integration apparatus 800 may communicate with components within the vehicle other than the components illustrated in FIG. 8. In addition, the vital integration device 880 may additionally receive network data items and the like from the outside of the vehicle.

The vital data integration device 800 may receive a plurality of different vital data items from a plurality of cameras 220, respectively, within the vehicle, for example, a first camera 221, a second camera 222, and a third camera 223.

The plurality of cameras 220 may be installed at different positions, respectively, within the vehicle. For example, the first camera 221 is an IR camera installed on an interior rearview mirror of the vehicle, and the second camera 222 may be an A-pillar camera. In addition, the third camera 223 may be an IR camera installed in the cluster. However, the plurality of cameras 220 are not limited to being installed at these positions and may be installed at other positions within the vehicle 100 and may include more than three cameras.

The plurality of cameras 220 acquire the vital data items of the driver or occupant using a remote PPG (rPPG) technique, respectively. For example, the plurality of cameras 220 may respectively capture facial images of the driver or occupant. Then, the plurality of cameras 220 may collect heart rate data items of the driver or occupant using the remote PPG (rPPG) technique, based on changes in blood flow, the change being measured by applying the face recognition algorithm to each of the acquired facial images. In this case, it should be noted that the plurality of vital data items acquired by the plurality of cameras 220, respectively, are heart rate data items of the same individual.

The vital data integration apparatus 800 may receive a detection result and/or a control result from at least one of the following: the motion detection module 235, the ADAS system 300, or the vehicle control unit 170, and may evaluate the vital data item that varies with the detection result and/or the control result.

The respective reliabilities of the vital data items are quantitatively evaluated by taking into consideration parameters, such as the accuracy, currency, and consistency of each of the vital data items. For example, the first camera 221 provides a vital data item characterized by high accuracy and low currency, the second camera 222 provides a vital data item characterized by somewhat lower accuracy and high currency, and the third camera 223 provides a vital data item characterized by both medium accuracy and medium currency. In this case, as a result of the reliability evaluation, different weight values may be assigned to the vital data items, respectively, that are collected by these cameras.

The vital data integration device 800 may reflect, in the reliability evaluation, the detection result and/or the control result from at least one of the following: the motion detection module 235, the ADAS system 300, or the vehicle control unit 170 within the vehicle 100.

In an embodiment, the vital data integration device 800 may change respective weight values of received vital data items based on the detection results and/or the control results received from the motion detection module 235, the ADAS system 300, and/or the vehicle control unit 170.

For example, in a case where an event is detected by the ADAS system 300 and emergency braking is activated without the driver's intervention, the heart rate of the driver or occupant is likely to increase, and therefore, a higher weight may be temporarily assigned to the received vital data items.

As another embodiment, the vital data integration device 800 may change the period for calculating the weight values to be applied to the received vital data items based on the detection results and/or the control results received from the motion detection module 235, the ADAS system 300, and/or the vehicle control unit 170.

For example, in a case where the control results from the vehicle control unit 170 are the same or show small changes, the driver's heart rate may decrease due to the driver's drowsiness. Accordingly, the period for calculating the weight values for the vital data items may be adjusted to be shorter, thereby enabling execution of a drowsiness prevention service.

As another embodiment, the vital data integration device 800 may adjust the degree of weight adjustment or the algorithm sensitivity for the vital data item based on the detection results and/or the control results received from the motion detection module 235, the ADAS system 300, and/or the vehicle control unit 170.

For example, while the movement of the driver or occupant is significantly detected according to the detection result from the motion detection module 235, the heart rate of the driver or occupant is likely to increase due to the physical movement of the driver or occupant. In this case, the degree of weight adjustment or the algorithm sensitivity for all the vital data items may be adjusted to be lower.

In an embodiment, the reliability evaluation of the vital data item by the vital data integration device 800 may also vary with a detection result and/or a control result from components within the vehicle 100 other than the components within the vehicle 100 illustrated in FIG. 8, or with network data items and the like from the outside of the vehicle 100. For example, the reliability evaluation of the vital data item of the driver or occupant may vary with traffic situation information, weather information, health state information received from the driver's or occupant's terminal, and other information.

In this manner, when weight values are assigned as a result of the reliability evaluation, among the vital data items to which weight values are respectively assigned, a final vital data item is generated by selecting a vital data item or fusing vital data items. The generated final vital data item is input to the state estimation algorithm for the driver or occupant. In this case, the finally generated vital data item is a reliable heart rate data item, and thus, the state of the driver or occupant may be estimated more accurately.

FIG. 9 is a block diagram that is referenced to describe a detailed configuration of the vital data integration device 800 according to the embodiment of the present disclosure.

With reference to FIG. 9, the vital data integration device 800 according to the present disclosure may include a reception unit 810, a data determination unit 820, and a data provision unit 830. In this case, the data determination unit 820 may perform the same function as a control unit or a processor. In addition, the data provision unit 830 may be a transmission unit or a transceiver for transferring the finally generated vital data item to the state estimation algorithm for the driver or occupant.

The receiver unit 810 may receive, as vital data items, heart rate data items derived from the remote PPG (rPPG) measured from facial images acquired by the plurality of cameras installed at different positions, respectively, within the vehicle 100.

The received vital data items correspond to a plurality of measured heart rate data items of the same individual. As an example, the plurality of cameras installed at different positions, respectively, within the vehicle 100 acquire the plurality of vital data items, respectively, of the same individual, but are not limited to using this data acquisition technique.

The plurality of vital data items received by the reception unit 810 have different values and different levels of precision. The reception unit 810 provides the vital data items to the data determination unit 820, and the data determination unit 820 selects a vital data item or integrates vital data items to provide a high-reliability driver state determination service.

The data determination unit 820 assigns respective weight values to the received vital data items by evaluating the respective reliabilities thereof. In addition, the data determination unit 820 selects a high-reliability vital data item or fuses a plurality of vital data items on the basis of the weight values assigned to the vital data items.

The data determination unit 820 may evaluate the respective reliabilities of the vital data items on the basis of information about landmarks contained in the facial image of the driver or occupant, the logic reliability, the amount of change compared with that of the previous vital data item, the respective reliabilities of the plurality of cameras, and the like. In this case, the evaluation result of the reliability is expressed as a weight value.

The data determination unit 820 may select or fuse one or more of the received vital data items on the basis of the information of landmarks contained in the facial image of the driver or occupant, the logic reliability, the amount of change compared with that of the previous vital data item, the respective reliabilities of the plurality of cameras, and the like. For example, the vital data item having an abnormal value may be removed, or the omitted vital data items may be ignored.

The data provision unit 830 receives the selected vital data item or the vital data item obtained by the fusion from the data determination unit 820.

The data provision unit 830 provides the finally generated vital data item to a state index estimation algorithm 840 for high-level vital analysis in such a manner that the state index of the driver or occupant is estimated on the basis of the selected vital data item or the vital data item obtained by the fusion. In this case, the state index of the driver or occupant may refer to one of the following: a stress index, a drowsiness index, or a fatigue index of the driver or occupant.

The state index estimation 840 calculates the fatigue index and the stress index and evaluates whether an emergency state of the driver or occupant occurs, by using the finally generated vital data item as the input data items to each state estimation algorithm. Various operations and services related to the travel and safety of the vehicle 100 may be provided to the vehicle 100 on the basis of the calculated fatigue index, the calculated stress index, and the result of evaluating whether the emergency state has occurred.

FIG. 10 is a representative flowchart that is referenced to describe a vital data integration method according to an embodiment of the present disclosure.

Each step illustrated in FIG. 10 may correspond to commands performed by a processor of a calculating apparatus. In this regard, the vital data integration method may be implemented in the form of a computer program recording medium including downloadable computer programs or commands.

Each step in the flowchart in FIG. 10, unless otherwise described, is performed by the above-described vital data integration device 800, more specifically, by any one of the following: the reception unit 810, the data determination unit 820, or the data provision unit 830, all of which are described with reference to FIG. 9.

The vital data integration device 800 receives respective vital data items measured by the plurality of cameras installed within the vehicle (S10).

The plurality of cameras installed within the vehicle each acquires a heart rate of the driver or occupant as a vital data item using the remote RRG (rPPG) technique. The remote photoplethysmography (rPPG) technique refers to a technique for acquiring biometric signals in a non-contact manner. Specifically, the remote photoplethysmography (rPPG) technique refers to a technique for extracting skin pixels from the facial image acquired by the camera, analyzing changes in skin color due to blood flow caused by a heartbeat, and acquiring a heart rate data item.

When a vital data item is received, the vital data integration device 800 assigns respective weight values to the received vital data items by evaluating the respective reliabilities thereof (S20).

In an embodiment, Step S20 of assigning the respective weight values may include a step of evaluating the respective reliabilities of the vital data items and determining different weight values thereof on the basis of at least one of the following: the information about landmarks in the images captured by the plurality of cameras, the areas occupied by the faces in the images, or the amount of change compared with that of the previous vital data item.

For example, different weight values may be assigned to the cameras, respectively, on the basis of the accuracy of the face recognition algorithm used in each of the plurality of cameras. In addition, for example, a higher weight may be assigned to a vital data item derived from an acquired facial image in which many landmarks appear or from an image in which the occupant covers a large area. Alternatively, for example, a higher weight may be assigned to a vital data item derived from an image in which the angle between the surface of the face and the optical axis of the camera is close to a right angle. In addition, for example, a higher weight may be assigned to a vital data item derived from an image that exhibits a high value of accuracy among output values of the face recognition algorithm. In addition, for example, a higher weight may be assigned to a vital data item that exhibits high continuity and similarity when the amount of change thereof is compared with that of the previous vital data item.

In an embodiment, Step S20 of assigning the respective weight values may include a step of additionally adjusting the respective reliability weight values of the vital data items based on control information of the vehicle and movement information of the occupant.

In this case, the control information of the vehicle refers to control information for controlling braking, acceleration, and steering of the vehicle. In this case, the braking, acceleration, and steering of the vehicle include sudden braking, sudden acceleration, and sudden steering executed by the driver's operation or executed by the operation of the ADAS system without the driver's intervention.

In addition, in this case, the movement information of the occupant refers to the result of monitoring the movement of the driver or occupant, obtained through the operation of a camera-based motion monitoring module.

In this manner, after different weight values are respectively assigned to the vital data items, the vital data integration device 800 selects a vital data item or fuses vital data items on the basis of the assigned weight values (S30).

The selection of a vital data item or the fusion of vital data items may be performed using one of the following: 1) a data fusion technique, 2) a soft voting technique, or 3) a combination of the soft voting technique and the data fusion technique.
1) The data fusion method refers to a method of removing an abnormal vital data item from among a plurality of measured vital data items and then providing a single data stream into which the respective values of vital data items are integrated. In this case, the abnormal vital data item refers to a vital data item that has a different value than the other vital data items or has a value representing a large amount of change.
2) The soft voting technique calculates a weighted probability for each of the measured vital data items by averaging the respective probability values of the measured vital data items. The soft voting technique refers to a technique for finally selecting a label with the highest probability among a plurality of calculated weighted probabilities.
3) The combination of the soft voting technique and the data fusion technique involves performing the data fusion technique after performing the soft voting technique. The data fusion technique is additionally performed, in which the respective weighted probabilities of the measured vital data items are calculated by averaging the respective probability values of the measured vital data items to derive a weighted average on the basis of the respective probability values.

The vital data integration device 800 selects a vital data item or fuses vital data items and finally generates a reliable vital data item.

Next, the vital data integration device 800 may provide the finally generated vital data item as an input data item for the state index estimation 840 (in FIG. 9), thereby enabling estimation of a state index of the driver (S40).

In this case, the finally generated vital data item may be provided simultaneously or sequentially to a plurality of different state index algorithms, and various state indices for the same individual may be provided as result values.

In some embodiments, the vital data integration device 800 may provide the finally generated vital data item to a device, a server, a cloud server, and the like that include various state index algorithms. In this case, the state index estimation 840 may be configured to be positioned outside the vital data integration device 800. Subsequently, the result values of the state indices may be transmitted to the vital data integration device 800 or the vehicle 100 and may be used to track the state of the driver or occupant and/or provide an associated service.

As described above, the vital data integration method according to the embodiment of the present disclosure may enable seamless and reliable data collection for analyzing and tracking the state of the driver or occupant. That is, a reliable input data item may be generated and provided for high-level vital data analysis.

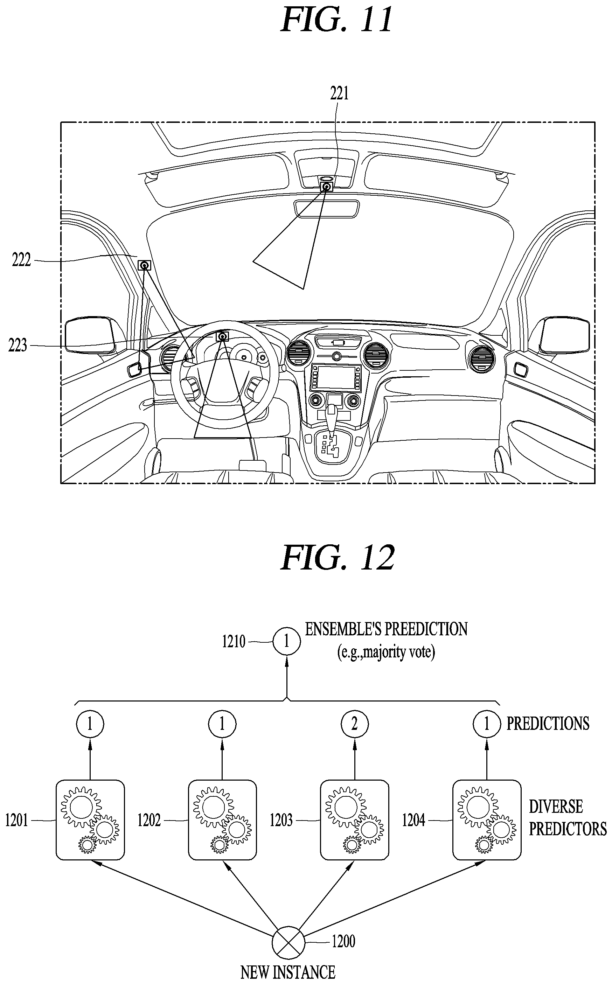

FIG. 11 is a view that is referenced to describe the collection of vital data items using the plurality of cameras within the vehicle associated with the embodiment of the present disclosure.

With reference to FIG. 11, for example, the vital data items of the driver are acquired using three cameras around the driver's seat within the vehicle 100. As illustrated, the plurality of cameras may include the IR camera (the first camera) 221 installed on the interior rearview mirror, the A-pillar camera (the second camera) 222 on the window side of the driver's seat, and the IR camera (the third camera) 223 installed in the cluster.

However, the number of cameras illustrated in FIG. 11 and the respective positions thereof may vary according to embodiments. Even in the case of such variation, it should be noted that the plurality of vital data items of the same individual are acquired by the plurality of cameras, from which a vital data item is selected or vital data items are fused.

The plurality of cameras, for example, the cameras 221, 222, and 223, acquire the vital data items of the driver or occupant, respectively, using the remote PPG (rPPG) technique. However, the plurality of cameras are not limited to using the remote PPG (rPPG) technique and may also acquire camera-based vital data items using other techniques.

In FIG. 11, the plurality of cameras, for example, the cameras 221, 222, and 223, are installed around the driver's seat within the vehicle and measure the vital data items of the driver using the Remote PPG (rPPG) technique. The plurality of cameras, for example, the cameras 221, 222, and 223, measure different vital data items, respectively, at the same time (or at short time intervals).

Specifically, the plurality of cameras, for example, the cameras 221, 222, and 223 measure heart rate data items in accordance with changes in blood flow, respectively, from the facial image of the driver or occupant seated in the driver's seat within the vehicle, and transmit the acquired heart rate data items to the vital data integration device 800 of the vehicle. The measured vital data items have different values and different levels of precision.

The measured vital data items are temporally synchronized in the reception unit 810 (in FIG. 9) of the vital data integration device 800 and are transferred to the data determination unit 820.

As described above, since the plurality of vital data items, measured at the same time, have different values and different levels of precision, data selection or data fusion (integration) is necessary to provide a high-reliability driver state determination service.

FIG. 12 is an exemplary view that is referenced to describe the data selection that uses a hard voting technique according to an embodiment of the present disclosure. The hard voting technique is also referred to as a majority voting technique in that the result that receives the highest number of votes is selected from among the respective results of the models.

With reference to FIG. 12, when a new driver state instance 1200 is captured through a plurality of means for sensing the state of the driver, for example, cameras 1201, 1202, 1203, and 1204, predicted states of the driver, which are associated with the driver state instance 1200, are respectively sensed by the cameras 1201, 1202, 1203, and 1204. As a result, when it is assumed that the respective sensing results of the three cameras 1201, 1202, and 1204 are predicted as '1,' and the sensing result of the remaining camera 1203 is predicted as '2,' '1' is finally selected, as a final data item 1210, according to the majority rule, that is, the ensemble's prediction.

The soft voting technique used according to the present disclosure yields more accurate and flexible results than the above-described hard voting technique. The soft voting technique is also referred to as a 'probability voting' technique. Specifically, the soft voting technique provides a result as a probability value without disregarding the sensing result of the specific camera 1203, which is illustrated in FIG. 12.

Specifically, it is assumed that three camera sensors measure the heart rate of the driver and that each sensor measures the heart rate of the driver every second. At this point, the soft voting technique determines the heart rate of the driver by performing the following process.

The third camera sensors transmit both the measured values and the time information reliability. The vital data integration device 800 temporally synchronizes the heart rate data items respectively transmitted from the cameras, and integrates the temporally synchronized heart rate data items into a single data stream.

For example, it is assumed that Camera A measures 80 bpm at 10:00:01, Camera B measures 82 bpm at 10:00:02, and Camera C measures 78 bpm at 10:00:03. The vital data integration device 800 integrates the heart rate data items as a single data stream and performs the task of removing the previously missing or abnormal value.

Specifically, the data determination unit 820 of the vital data integration device 800 removes a vital data item having an abnormal value among the plurality of vital data items, and then evaluates the respective reliabilities of normal vital data items to assign respective weight values thereto. At this point, the respective reliabilities of the normal vital data items are quantitatively evaluated by taking into consideration parameters such as the accuracy, currency, and consistency of the normal vital data item.

For example, as described above, it is assumed that Camera A provides a vital data item characterized by high accuracy and low currency, Camera sensor B provides a vital data item characterized by low accuracy and high currency, and Camera C provides a vital data item characterized by both medium accuracy and medium currency. In this case, different weight values may be assigned to these vital data items, respectively.

After different weight values are assigned in this manner, the vital data integration device 800 may also select an optimal vital data item or an average value according to the reliability of each vital data item (the data fusion technique). Furthermore, the vital data integration device 800 may calculate a weighted probability value by combining (fusing) the vital data items, and then select a label with the highest probability (the soft voting technique). Alternatively, the vital data integration device 800 may select the average of the weighted probability values (the soft voting technique + the data fusion technique).

The reliable data item that is finally generated in this manner may be provided for the index estimation algorithm 840 (in FIG. 9), and thus, a more accurate and reliable state index result may be acquired.

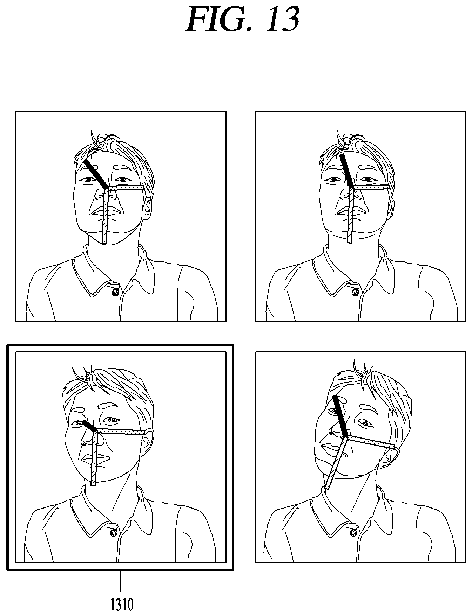

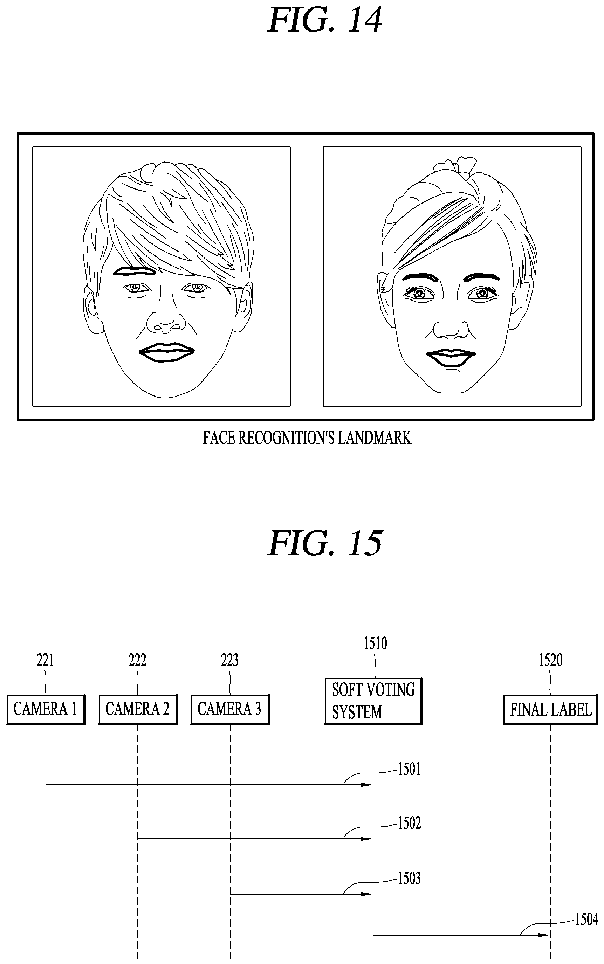

FIGS. 13 and 14 are views that are referenced to describe examples in which a face recognition algorithm according to an embodiment of the present disclosure assigns different weight values to vital data items, respectively.

In an embodiment of the present disclosure, the reliability evaluation of vital data items (e.g., heart rate data items) received from the plurality of cameras is performed by determining the respective weight values of the vital data items on the basis of at least one of the following: the respective reliabilities of the plurality of cameras, the information about landmarks in the images captured by the plurality of cameras, the areas occupied by the faces in the images, the logic reliability, or the amount of change compared with that of the previous vital data item.

The vital data items measured by the plurality of cameras are acquired at the same time but have different levels of precision. Accordingly, the data selection or data fusion (integration) is necessary to provide the high-reliability driver state determination service.

According to the present disclosure, the data fusion technique and the soft voting technique are used as data processing techniques for selecting a vital data item or fusing vital data items from among the vital data items to which weight values are respectively assigned.

The data fusion technique is a data processing technique in which respective values, which are obtained by applying respective weight values to the results of applying the face recognition algorithm to the facial images captured by the plurality of cameras, are integrated into a single data stream, thereby acquiring a high-accuracy and flexible result.

The soft voting technique is a data processing technique in which respective weight values are assigned to the results of applying the face recognition algorithm to the facial images captured by the plurality of cameras, the respective probability values of the measured vital data items are averaged, and a label with the highest probability is finally selected.

Before the data selection or data fusion, a technique in which respective weight values are assigned to vital data items is as follows. The assignment of respective weight values to the vital data items begins with determining to which factors the respective weight values are assigned. Specific examples in which weight values are assigned on the basis of various factors will be described below.
1) In a case where the result of applying the face recognition algorithm is reflected as a factor:
   In an embodiment, a weight may be assigned to an image in which the angle between each optical axis of the plurality of cameras and the surface of the face of the driver or occupant is close to a right angle.

The angle of the surface of the face of the driver or occupant may be ascertained when the face recognition algorithm within the deep learning algorithm is executed. The angle of the surface of the face of the driver or occupant affects a change in skin color that varies with a change in blood flow. The heart rate (HR) is measured from the change in skin color, and the landmark size is determined based on the angle of the face. For this reason, a weight is assigned to the image, captured by the camera, in which the angle between the surface of the face of the driver or occupant and the optical axis of the camera is close to a right angle.

For example, with reference to FIG. 13, the plurality of cameras installed within the vehicle 100 have respective image capture angles that vary with the head movement of the driver or occupant at every image capture time. Accordingly, as illustrated in FIG. 13, facial images are captured at various image capture angles at every image capture time. In FIG. 13, a weight is assigned to an image 1310 in which the image capturing angle between the optical axis of the camera for image capturing and the surface of the face of the driver or occupant is close to a right angle.

In another embodiment, a high weight may be assigned to a facial image in which landmarks are largest and most distinct among facial images obtained through the plurality of cameras.

The significantly important factors when executing the face recognition algorithm within the deep learning algorithm are the extraction of facial landmarks and the determination of the area thereof. The reason for this is that the size of the facial landmark significantly affects the performance of the algorithm subsequent to face recognition.

The facial landmark refers to a facial feature point, such as an eye, a nose, a mouth, or an eyebrow. When images are captured through the plurality of cameras, for example, the face detection algorithm detects the face of the driver or occupant, and subsequently, a facial landmark algorithm may detect landmarks (e.g., the eyes, the nose, the mouth, the eyebrows, and the like) within the face.

From FIG. 14, it can be seen that the landmarks such as the eyes and the eyebrows are detected as a result of applying the facial landmark algorithm. Among a plurality of images captured at the same time by the plurality of cameras, as illustrated in FIG. 14, a weight is assigned to an image, for use as the main image, in which the facial landmark of the driver or occupant appears largest and most distinct.

In another embodiment, among the plurality of images captured by the plurality of cameras, a weight may be assigned to an image in which many facial landmarks appear.

In another embodiment, among the plurality of images captured by the plurality of cameras, a weight may be assigned to an image in which the driver or occupant covers a large area.

Specifically, in this technique, before landmarks are detected, prior to the application of the deep learning algorithm, a weight is assigned to an image in which the face and body of the driver or occupant cover large areas. In this technique, in a case where the driver or occupant wears items such as a hat or sunglasses that obscure the face, although the landmarks may be limited, a weight is assigned to an actual image in which the driver or occupant covers a large area. This technique, unlike a face landmark technique, differs in that a significant portion of the outline of the driver or occupant is utilized differently.

As another embodiment, among the plurality of cameras, a weight may be assigned to a camera in which a higher-accuracy algorithm is executed.

The algorithms executed in the plurality of cameras may vary in performance. In addition, the same rPPG algorithm may differ in optimization depending on the installation position, performance, and type of the camera, thereby causing the algorithm to differ in performance. For example, in a case where the algorithm of Camera A has an accuracy of 95% and where the algorithm of Camera B has an accuracy of 75%, a higher weight may be assigned to Camera A.

As another embodiment, among resultant values derived from the same algorithm, a weight may be assigned to the resultant value with a higher accuracy.

When an algorithm for measuring a vital data item is executed, data accuracy, indicating an accuracy state of the currently measured vital data item, is output as a resultant value. Accordingly, for the utilization in the fusion of vital data items or the selection of a vital data item, a weight may be assigned based on the accuracy included in the resultant value of this algorithm.

As another embodiment, among the plurality of vital data items, a weight may be assigned to a vital data item, the value of which is highly continuous with and similar to the value of the previous vital data item.

The vital data items of a person have continuity. That is, in a case where a specific index increases or decreases in vital data items, the specific index tends to vary linearly. Based on these characteristics, in a case where a vital data item changes rapidly as compared with the previous vital data item, there is a high probability that the corresponding vital data item is an outlier or that a measurement error has occurred. Accordingly, in a case where a successively measured vital data item loses linearity or exhibits excessive volatility, a higher weight may be assigned to a vital data item exhibiting high similarity to the previous vital data item, thereby allowing the evaluation of the data reliability.

For example, it is assumed that when the heart rate is measured every second, Camera A measures 60 bpm at 0 seconds, 75 bpm at 1 second, and 61 bpm at 2 seconds. In addition, it is assumed that when the heart rate is measured every second, Camera B measures 60 bpm at 0 seconds, 62 bpm at 1 second, and 61 bpm at 2 seconds. Then, a weight may be assigned to the result of the vital data item measured by Camera B, the result exhibiting continuity with the previous heart rate data at 1 second, thereby ensuring data reliability.

2) In a case where the performance of the camera itself is reflected as a factor:
As an embodiment, a weight may be assigned to the camera that captures an image in which the facial portion has a high pixel count. This is to reflect the property that as the number of pixels of the camera increases, the facial landmarks become clearer and larger.

As another embodiment, a weight may be assigned to the camera that exhibits a high resolution. This is to reflect the property that as the resolution of the camera increases, the facial landmarks have respective clear and larger areas.

As another embodiment, a weight may be assigned to the camera that has a high frame rate.

The frame rate of the camera significantly affects the accuracy of estimating the heart rate (HR) after measuring a vital data item or calculating the stress index, which is one of the vital post-processing operations. Accordingly, a high frame rate is essential for more accurate processing of vital data items. The plurality of cameras installed in the vehicle 100 have a significant difference in frame rate depending on the purpose of installation and the performance of an ISP processing chip. Accordingly, a weight may be assigned to the magnitude of the frame rate.

As another embodiment, a weight may be assigned based on a high overall brightness or a partial brightness of an image captured by the camera.

When rPPG is measured using an RGB camera, algorithm performance may also be significantly susceptible to shadows or light cast on the face of the driver or occupant within the vehicle. In addition, when the surroundings are too bright or too dark, the algorithm may not be executed at all. This is the same for an IR camera, as well as an RGB camera. Specifically, the IR camera is susceptible to disturbance light. Therefore, the image illumination is a factor that significantly affects the performance of the algorithm. Accordingly, the algorithm performance may be improved by assigning a specific weight to high overall illumination or high local illumination of an image captured by the camera.

As another embodiment, a weight may be assigned to low noise or the like within an image. The noise within the image is a primary cause of reducing the accuracy of the algorithm. Accordingly, a low weight may be assigned to an image within which the noise is high, thereby allowing an improvement in algorithm performance.

The factors to which a weight is assigned in evaluating the respective reliabilities of the vital data items acquired by the plurality of cameras have been described above. The technique in which a weight is assigned to the factors will be described in detail below.

A technique in which vital data items are measured in a non-contact manner through the plurality of cameras within the vehicle 100 requires a calibration operation and error removal operations for the vital data items because measurement errors occur during the measurement.

According to the present disclosure, a technique is proposed in which respective weight values of measured vital data items are assigned based on the evaluation thereof, thereby allowing processing of the vital data items. According to the present disclosure, as described above, 1) the data fusion technique, 2) the soft voting technique, and 3) the combination of the data fusion technique and the soft voting technique, which are techniques of distributing respective weight values to vital data items, are used.

The data fusion technique is a data processing technique in which vital data items are acquired in real time from the plurality of cameras capturing an image of the driver or occupant, the vital data items acquired by the plurality of cameras are temporally synchronized, and the plurality of vital data items are integrated into a single data stream.

The soft voting technique is a data processing technique in which a final vital data item is generated by averaging normal vital data items, among vital data items acquired by each of the plurality of cameras, or by integrating the normal vital data items via soft voting, excluding omitted or abnormal vital data items.

The vital data item finally generated through the data fusion technique, the soft voting technique, or the combination of the data fusion technique and the soft voting technique is transferred to an upper-level layer, and is used as an input data item for estimating the state index of the driver or occupant.

FIG. 15 is a flowchart that is referenced to describe a method, according to an embodiment of the present disclosure, of generating a final input data item by selecting a vital data item or fusing vital data items via the soft voting technique.

First, the plurality of cameras within the vehicle 100, for example, the first camera 221, the second camera 222, and the third camera 223 measure vital data items, respectively, of the same driver or occupant at the same time.

Specifically, the vital data item measured by the first camera 221 is transmitted to a soft voting system 1510. In addition, the vital data measured by the second camera 222 is transferred to the soft voting system 1510. In addition, the vital data measured by the third camera 223 is transferred to the soft voting system 1510.

The soft voting system 1510 may be included in the form of an algorithm, a processor, or a program within the vital data integration device 800. Alternatively, the soft voting system 1510 may be the data determination unit 820 (in FIG. 9) of the vital data integration device 800. The soft voting system 1510 is disclosed below as the data determination unit 820.

The data determination unit 820 selects a vital data item having a high reliability weight on the basis of the respective weight values assigned to the vital data items, or fuses vital data items to which respective reliability weight values are assigned, and transfers the selected vital data item or the vital data item obtained by the fusion to the data provision unit 830 (in FIG. 9).

The data determination unit 820 applies respective weight values to the respective probability values of the vital data items and then calculates an average on the basis of the assigned weight values. Subsequently, the data determination unit 820 calculates the respective reliability-weighted probabilities of the vital data items.

In another embodiment, the data determination unit 820 calculates the average of the respective weight values of the vital data items on the basis of the weight values assigned to the vital data items, selects vital data items based on the calculated average, integrates the selected vital data items into a single stream (which is referred to as 'data fusion'), and transfers the single stream to the data provision unit 830.

In another embodiment, the data determination unit 820 may apply a weight to a probability value of each vital data item and then select a vital data item having the highest probability (the label with the highest probability) among the reliability-weighted probabilities. In addition, the data determination unit 820 may generate a vital data item obtained by the fusion by calculating the average of the reliability-weighted probabilities and transmit the selected vital data item or the vital data item obtained by the fusion to the data provision unit 830.

Next, the finally generated reliable vital data item is provided to a final label 1520 (1504), thereby allowing more accurate estimation of the state index of the driver or occupant.

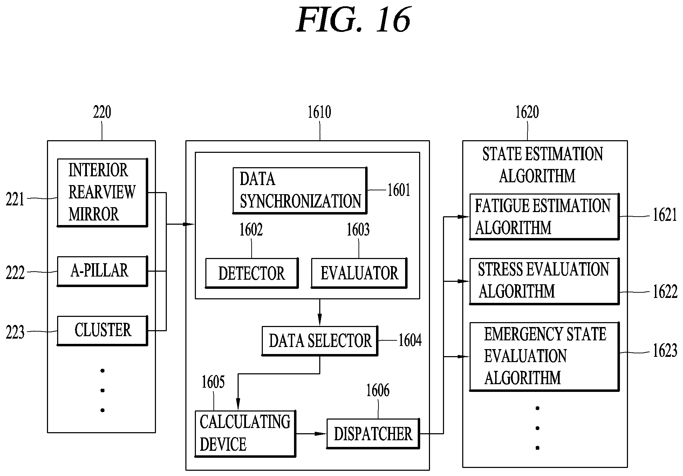

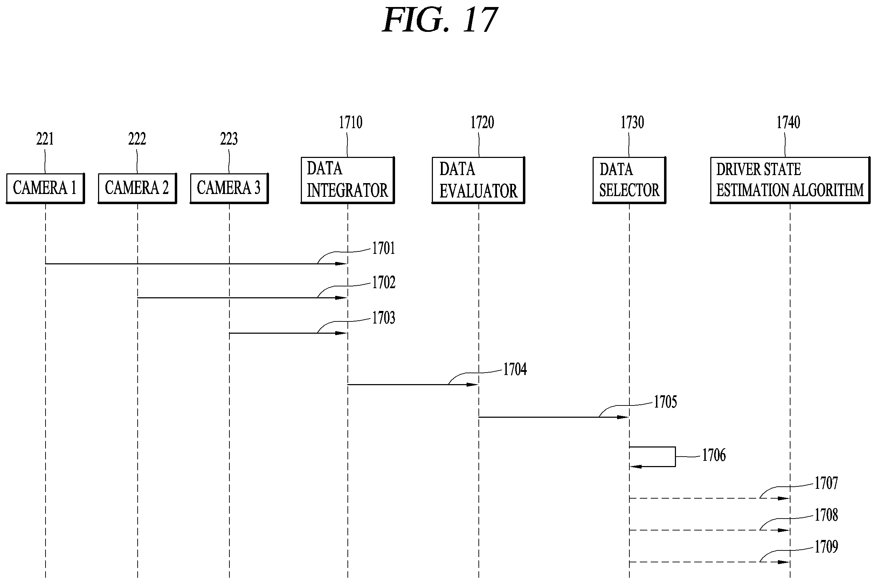

FIGS. 16 and 17 are a block diagram and a flowchart that are referenced to describe a process in which, according to an embodiment of the present disclosure, a plurality of vital data items are acquired, and then vital data items are integrated, or a vital data item is selected to be provided to the state estimation algorithm for the driver.

With reference to FIG. 16, the plurality of cameras 220 may include the first camera 221 installed on the interior rearview mirror, the second camera 222 on the driver seat side of the A-pillar, and the third camera 223 installed in the cluster.

A plurality of vital data items acquired at the same intervals by the plurality of cameras 220 are transferred to a vital data analysis module 1610. In this case, the vital data analysis module 1610 may refer to the vital data integration device 800 according to the present disclosure.

The vital data analysis module 1610 may include a data synchronizer 1601, a detector 1602, an evaluator 1603, a data selector 1604, a calculating device 1605, and a dispatcher 1606.

The data synchronizer 1601 temporally synchronizes, substantially at the same time, the plurality of vital data items measured by the plurality of cameras.

The detector 1602 may remove an omitted vital data item or a vital data item having an abnormal value from among the temporally synchronized vital data items and detect vital data items having normal values.

The evaluator 1603 evaluates reliability according to a preset reliability reference for each vital data item and assigns a weight thereto.

For example, a technique may be adopted and applied in which a weight is assigned according to the preset reliability reference in such a manner that the angle between the surface of the face and the optical axis of the camera is close to a right angle.

When it is assumed that each of the plurality of cameras 220 image-captures the same driver or occupant A, the closer the image-capturing angle is to a right angle, the higher the assigned rank or weight is. As an example, a weight of '3' is assigned to the first camera 221 having the image-capturing angle closest to a right angle, a weight of '2' is assigned to the second camera 222, and a weight of '1' is assigned to the third camera 223.

The data selector 1604 and the calculating device 1605 select a final vital data item or fuse vital data items based on the respective weight values assigned to a plurality of vital data items, and provide the selected vital data item or the vital data item obtained by the fusion to the dispatcher 1606.

Specifically, the data selector 1604 and the calculating device 1605 may calculate the weighted average of the plurality of vital data items as a new final vital data item based on the respective weight values assigned to the plurality of vital data items. For example, the final data item for the same driver or occupant A in the above example may be calculated as a value corresponding to one-third of a sum of the value of the first camera 221 multiplied by 3, the value of the second camera 222 multiplied by 2, and the value of the third camera 223 multiplied by 1.

As another example, the data selector 1604 and the calculating device 1605 may finally select a label with the highest probability by averaging the probability values based on the weight values assigned to the plurality of vital data items or generate a new final vital data item by calculating the weighted average of the values of the plurality of vital data items.

Specifically, in the above example, it is assumed that the first camera 221 has a recognition probability of 0.8 and a weight of 0.6, the second camera 222 has a recognition probability of 0.7 and a weight of 0.3, and the third camera 223 has a recognition probability of 0.6 and a weight of 0.1. These weight values are arranged in decreasing order as the corresponding image-capturing angle deviates from a right angle.

In this case, the weighted probability values on a per-camera basis are as follows. Specifically, the weighted probability value of the first camera 221 is calculated as (0.8 x 0.6)/(0.6 + 0.3 + 0.1) = 0.48. The weighted probability value of the second camera 222 is calculated as (0.7 x 0.3)/(0.6 + 0.3 + 0.1) = 0.21. The weighted probability value of the third camera 223 is calculated as (0.6 x 0.1)/(0.6 + 0.3 + 0.1) = 0.06.

In this case, the vital data measured by the first camera 221, which has the highest weighted probability value, may be finally selected. Alternatively, the weighted average of the values of the vital data items may be determined as a single final value obtained by fusing the vital data items by additionally using the data fusion technique.

The dispatcher 1606 extracts a finally generated vital data item and provides the finally generated vital data item to a state estimation algorithm 1620.

The state estimation algorithm 1620 calculates or estimates various state indices using a reliable vital data item transferred from the dispatcher 1606 as an input data item.

As illustrated in FIG. 16, the input data item may be transferred to a fatigue estimation algorithm 1621, a stress evaluation algorithm 1622, and an emergency state evaluation algorithm 1623. Accordingly, the fatigue of the driver or occupant may be estimated, the stress index thereof may be calculated, and the presence or absence of the emergency state thereof may be evaluated. The results of evaluating the algorithms are transferred to the vehicle 100, thereby allowing an associated additional operation or service to be performed. Of course, in addition to the algorithms illustrated in FIG. 16, more state index algorithms may be provided.

FIG. 17 is a flowchart associated with the block diagram of FIG. 16.

With reference to FIG. 17, a vital data item measured by the first camera 221 is transferred to a data integrator 1710 (S1701). In addition, a vital data item measured by the second camera 222 is transferred to the data integrator 1710 (S1702). In addition, a vital data item measured by the third camera 223 is transferred to the data integrator 1710 (S1701).

The data integrator 1710 temporally synchronizes a plurality of vital data items and removes an omitted vital data item or a vital data item having an abnormal value, and then transfers the remaining temporally synchronized vital data items to a data integrator 1720 (S1704).

The data evaluator 1720 evaluates the respective reliabilities of the vital data items according to a preset reliability reference, thereby calculating the respective weight values thereof, and transfers the calculated weight values to a data selector 1730 (S1705).

The data selector 1730 selects a final reliable vital data item or fuses reliable vital data items according to a data processing technique based on the data fusion technique, the soft voting technique, or the combination of the data fusion technique and the soft voting technique, all of which are described above (S1706) The vital data item finally generated by the data selector 1730 is transmitted to various algorithms of a driver state estimation algorithm 1740 (S1707, S1708, and S1709). Accordingly, various state indices may be transferred to an associated operating system within the vehicle 100, thereby allowing the provision of various services.

In embodiments of the present disclosure, the vital data integration device 800 may reflect the result of measurement by another sensor and/or the result of control by the operating system within the vehicle in evaluating the respective reliabilities of the vital data items.

In an embodiment, the data determination unit 810 (in FIG. 9) of the vital data integration device 800 may perform the same function as a control unit or a processor.

The respective reliability weight values of the vital data items may be adjusted to vary with the control information of the vehicle and the movement information of the occupant.

In this case, the control information of the vehicle refers to control information for controlling braking, acceleration, and steering of the vehicle. In this case, the braking, acceleration, and steering of the vehicle include sudden braking, sudden acceleration, and sudden steering executed by the driver's operation or executed by the operation of the ADAS system without the driver's intervention.

In addition, the movement information of the occupant refers to the movement information of the driver or occupant acquired through the camera-based motion monitoring module, and is measured by the motion monitoring module installed in the vehicle 100.

In an embodiment, the data determination unit 820 of the vital data integration device 800 may determine, based on the control information of the vehicle, that the respective amounts of change in the vital data items have occurred temporarily. Furthermore, based on the determination, the data determination unit 820 may reduce the respective reliability weight values of the vital data items. Alternatively, in another embodiment, based on the determination, the data determination unit 820 may lengthen the period for calculating the respective weight values of the vital data items.

For example, a variation in the heart rate of the driver or occupant may be significantly affected by abrupt vehicle control, such as when a braking event occurs due to a traffic accident ahead during operation of the vehicle, or by an internal or external event in the vehicle 100. In this case, the period for calculating the weight values for the vital data items may be temporarily lengthened, or the magnitude of the weight resulting from evaluation may be reduced.

In another embodiment, the data determination unit 820 of the vital data integration apparatus 800 may determine that the control information of the vehicle, which is received for a predetermined time or more, remains unchanged. Based on the determination, the data determination unit 820 may increase the respective reliability weight values of the vital data items or shorten the period for calculating the reliability weight values.

In this case, the predetermined time may be a duration sufficient to determine that there is no driving operation due to the occurrence of the driver's drowsiness or emergency state. However, the predetermined time may vary depending on the driving state of the vehicle 100, the traveling time zone, the health state information of the driver, and the like.

For example, during steering and braking operations, which remain unchanged or have no significant change for a long period of time, the driver may experience a change in the vital data items, such as a decrease in heart rate due to drowsy driving or the like in the corresponding situation. In this case, a service for preventing the driver's drowsiness may be facilitated by increasing the respective weight values of all vital data items measured by the plurality of cameras within the vehicle 100 or shortening the period for calculating the weight values.

In another embodiment, the data determination unit 820 of the vital data integration device 800 determines, based on the movement information of the occupant, that the respective amounts of change in the vital data items have occurred temporarily. Furthermore, according to the determination, the data determination unit 820 may reduce the respective reliability weight values of the vital data items or lengthen the period for calculating the reliability weight values.

For example, in a case where the driver eats or converses during operation of the vehicle or executes excessive movements involved in driving operations, such as steering maneuvers requiring a large steering angle, these physical movements may temporarily increase the heart rate of the driver. The heart rate variations caused by these temporary physical movements may cause confusion in determining the stress index or fatigue level of the driver and providing a service related to the driver state determination. Accordingly, in a case where a large movement of the driver or occupant is temporarily detected, the respective weight values of all vital data items measured by the plurality of cameras within the vehicle 100 may be reduced and/or the period for calculating the weight values may be lengthened.

As described above, the vital data integration device for the vehicle according to the embodiment of the present description can collectively provide vital data items of the driver or occupant in a stable, seamless, and highly precise manner when estimating the drowsiness, stress, health information, and the like of the driver or occupant. That is, the reliable input data items can be provided for high-level analysis of vital data items.

The present disclosure may be implemented in the form of computer-readable code recorded on a program-recorded medium. Computer-readable media include all types of recording devices capable of storing data that are readable by a computer system. Examples of computer-readable media include hard disk drives (HDDs), solid-state disks (SSDs), silicon disk drives (SDDs), ROM, RAM, CD-ROM, magnetic tapes, floppy disks, optical data storage devices, and other similar media. In addition, these media may further include transmission media, such as waves (e.g., waves transmitted over the Internet). In addition, the computer may include a controller or processor constituting the vital data integration device 800. Therefore, the detailed description should not be construed as limiting in any respect, but should be regarded as illustrative in nature. The scope of the present disclosure should be determined by the proper interpretation of the following claims. All equivalent modifications to the embodiments of the present disclosure fall within the scope of the present disclosure.

## Claims

1. A vital data integration device for a vehicle, the device comprising:
a reception unit that receives respective vital data items collected by a plurality of cameras installed within the vehicle;
a data determination unit that assigns respective weight values to the received vital data items by evaluating the respective reliabilities thereof, and selects a vital data item or fuses vital data items on the basis of the assigned weight values; and
a data provision unit that provides a finally generated vital data item to estimate a state index of a driver.

2. The vital data integration device of claim 1, wherein said evaluating the respective reliabilities of the received vital data items comprises determining different weight values of the received vital data items on the basis of at least one of the following: the respective reliabilities of the plurality of cameras, information about landmarks in images captured by the plurality of cameras, areas occupied by the faces in the images, or the amount of change compared with a previous value of the vital data item,.

3. The vital data integration device of claim 1, wherein the plurality of cameras are installed around a driver seat within the vehicle and measure a vital data item of the driver using a rPPG (remote PPG) method, and
wherein the reception unit temporally synchronizes a plurality of measured vital data items and transfers the resulting vital data items to the data determination unit.

4. The vital data integration device of claim 3, wherein the data determination unit removes a vital data item having an abnormal value from among the plurality of vital data items, then evaluates the respective reliabilities of normal vital data items, and assigns respective weight values to the normal vital data items.

5. The vital data integration device of claim 1, wherein the data determination unit selects a vital data item having a high reliability weight on the basis of the assigned weight values or fuses vital data items to which respective reliability weight values are assigned, and transfers the selected vital data item or the vital data item obtained by the fusion to the data provision unit.

6. The vital data integration device of claim 5, wherein the data determination unit calculates the average of the respective weight values of the vital data items on the basis of the assigned weight values, selects vital data items based on the calculated average, fuses the selected vital data items into a single stream, and transfers the single stream to the data provision unit.

7. The vital data integration device of claim 5, wherein the data determination unit applies the assigned weight values to the respective probability values of the vital data items, then calculates an average on the basis of the assigned weight values, and calculates the respective reliability-weighted probabilities of the vital data items.

8. The vital data integration device of claim 7, wherein the data determination unit selects a vital data item having the highest probability among the reliability-weighted probabilities or generates a vital data item obtained by the fusion, by calculating the average of the reliability-weighted probabilities, and transfers the selected vital data item or the vital data item obtained by the fusion to the data provision unit.

9. The vital data integration device of claim 1, wherein the data determination unit adjusts the respective reliability weight values of the vital data items based on control information of the vehicle and movement information of an occupant.

10. The vital data integration device of claim 9, wherein the data determination unit determines, based on the control information of the vehicle, that the respective amounts of change in the respective vital data items have occurred temporarily and, based on the determination, reduces the respective reliability weight values of the vital data items.

11. The vital data integration device of claim 10, wherein the control information of the vehicle is a control result that corresponds to any one of the following: braking, starting, or steering activation, each of which exceeds a range set by a driver's operation or an ADAS system.

12. The vital data integration device of claim 9, wherein the data determination unit determines that the control information of the vehicle, which is received for a predetermined time or more, remains unchanged, and, based on the determination, increases the respective reliability weight values of the vital data items or shortens the period for calculating the reliability weight values.

13. The vital data integration device of claim 9, wherein the data determination unit determines, based on the movement information of the occupant, that the respective amounts of change in the vital data items have occurred temporarily and, based on the determination, reduces the respective reliability weight values of the vital data items or lengthens the period for calculating the reliability weight values.

14. A vital data integration method for a vehicle, the method comprising:
receiving respective vital data items collected by a plurality of cameras installed within the vehicle;
assigning respective weight values to the received vital data items by evaluating the respective reliabilities thereof;
selecting a vital data item or fusing vital data items on the basis of the assigned weight values; and
estimating a state index of a driver by providing a finally generated vital data item as input data.

15. The method of claim 14, wherein the assigning of the respective weight values comprises determining different weight values of the received vital data items on the basis of at least one of the following: the respective reliabilities of the plurality of cameras, information about landmarks in images captured by the plurality of cameras, the areas occupied by the faces in the images, or the amount of change compared with a previous value oof the vital data item.

16. The method of claim 14, wherein the assigning of the respective weight values includes additionally adjusting the respective reliability weight values of the vital data items based on control information of the vehicle and movement information of an occupant.
